# EUROPEAN PATENT APPLICATION

(11) **EP 1 930 415 A1**
(43) Date of publication of application: **11.06.2008**
(21) Application number: 06811356.2
(22) Date of filing: 29.09.2006
(51) Int. Cl.: C12N 5/06, A61K 35/24, A61P 37/02, C12Q 1/02, G01N 33/15, G01N 33/50

(54) **DEVELOPMENT OF METHOD FOR SCREENING OF COMPOUND CAPABLE OF IMPROVING THE PRODUCTION OF REGULATORY T CELL, AND METHOD FOR PRODUCTION OF REGULATORY T CELL USING IMMUNOSUPPRESSIVE MACROLIDE ANTIBIOTIC**

(30) Priority: 30.09.2005 JP 2005289224
(71) Applicant: Kyoto University, Kyoto-shi, Kyoto 606-8501 (JP)
(72) Inventor: KOSHIBA, Takaaki, Kyoto-shi Kyoto 6068501 (JP); ITO, Atsushi, Kyoto-shi Kyoto 6068501 (JP); SAKAGUCHI, Shimon, Kyoto-shi Kyoto 6068507 (JP); TANAKA, Koichi, Kyoto-shi Kyoto 6060005 (JP); WU, Yanling, Kyoto-shi Kyoto 6068501 (JP); NARUMOTO, Mayuko, Kyoto-shi Kyoto 6068501 (JP)
(74) Representative: Duckworth, Timothy John
(86) International application number: PCT/JP2006/320029
(87) International publication number: WO 2007/037544

(57) **Abstract**

The present invention provides a screening method for a compound capable of inducing regulatory T cells, comprising the following steps:
(1) a step for culturing CD4⁺ T cells having a naive phenotype in the presence of a test compound, and isolating T cells from the culture product;
(2) a step for evaluating the immunosuppressive function of the T cells isolated in the step (1);
(3) a step for obtaining the foregoing test compound as a compound capable of inducing regulatory T cells if the results of the evaluation in the step (2) show that the T cells isolated in the step (1) have immunosuppressive function.

The present invention also provides a method of producing regulatory T cells, comprising culturing CD4⁺ T cells having a naive phenotype in the presence of a rapamycin compound to obtain regulatory T cells. The regulatory T cell produced by the method can be used as an immunomodulator for the prophylaxis or treatment of the rejection in organ transplantation, an allergic disease, an autoimmune disease, a graft-versus-host disease (GVHD), infertility and the like.

## Description

### Technical Field

The present invention relates to a method for screening for a compound capable of inducing regulatory T cells, a method of producing regulatory T cells using a rapamycin compound, an immunomodulator, a method of producing the immunomodulator, a kit for producing regulatory T cells, an agent for inducing regulatory T cells and the like.

### Background Art

After organ transplantation, immunosuppressants are used to inhibit the rejection. However, immunosuppressants often induce infection, and the leading cause of the death of the patients after transplantation is infection. As evidenced above, in the current transplantation therapy, while the surgical technique has been established, rejection suppressive measures and anti-infection measures produce conflicts in the treatment strategy. Accordingly, it is an issue of urgency to simultaneously achieve both of them. Given such situation, the study of the mechanism of establishment of immune tolerance - the state where a transplanted organ sufficiently functions without using an immunosuppressant - and the method of actively inducing the immune tolerance is becoming the main pillar in the field of transplantation therapy. This means that the study of transplantation therapy has completed the level of organ preservation and improvement of surgical techniques, and clearly entered the second stage.

In recent years, there are findings rapidly accumulated at the animal experiment level that CD25⁺CD4⁺ regulatory T cells (regulatory T cell(s) is sometimes referred to as Treg(s)) plays an important role in the immune tolerance after organ transplantation. The present inventors have confirmed that the cells expand in patients with naturally established immune tolerance after living liver transplantation (Am J Transpl, 4, 2118, 2004). The present inventors have also shown that a skin graft is not rejected in the mouse system by employing a method including once separating regulatory T cells, culturing the cells in vitro in a donor antigen-specific manner and returning them into the body, a so-called cell adoptive immunotherapy (Int Immunol., 16, 1189-1201, 2004). From these, a method effective for actively inducing the immune tolerance in transplantation therapy in human is presumed to be in vitro expansion of regulatory T cells and transferring them into the patient, namely, cell adoptive immunotherapy. When regulatory T cells are expanded in vitro, maintenance of regulatory function thereof is indispensable for the cell adoptive immunotherapy (Nat Rev Immunol., Mar;3(3), 199-210, 2003), and some trials and errors have been made for in vitro expansion of human regulatory T cells in a high fold without losing the immunoregulatory function thereof.

Human CD25⁺CD4⁺ regulatory T cell has been reported to play an essential role in vivo in the immune tolerance to the endogenous autoantigen, and more recently, to play an essential role in the immune tolerance in transplantation after organ transplantation and establishment of pregnancy, and to be useful for the prophylaxis of GVHD (graft-versus-host disease) and allergy (Annu Rev Immunol., 22, 531-562, 2004 Nat Rev Immunol., 2, 389-400, 2002,Nat Rev Immunol., 3, 199-210, 2003., Blood, 103, 2410-2416, 2004, Lancet, 363, 608-615, 2004, Trends Immunol., 25, 563-565. 2004). In vivo, human Tregs are characterized by memory phenotype-CD45RO⁺ in addition to the property of the cell surface CD4⁺ and CD25^{high+}, FOXP3 expression, intracellular CTLA-4, cell surface GITR, TNFRII, and unresponsiveness/immunosuppressive function to autoantigen and alloantigen and the like (J Immunol., 167, 1245-1253, 2001, J Exp Med., 193, 1285-1294, 2001, J Exp Med., 193, 1295-1302, 2001, Blood, 98, 2736-2744, 2001, Eur J Immunol., 32, 1621-1630, 2002, Int Immunol., 16, 1643-1656, 2004, Exp Hematol., 32, 622-629, 2004). In experiment, when thymus cells free of CD25⁺CD4⁺ cells are adoptively transferred into an immunodeficient host, autoimmune diseases are developed (J Immunol., 162, 5317-5326, 1999), and in a certain rodent model, extirpation of thymus before transplantation prevents immune tolerance in transplantation mediated by Tregs (Transplantation, 76, 588-596, 2003). In agreement with these experimental evidences, CD25⁺CD4⁺ T cell expressing FOXP3 at a high level are present in human thymus (Blood, 102, 4107-4114, 2003, Eur J Immunol, 35, 383-390, 2005).

The present inventors reported that CD45RA⁺CD25⁺CD4⁺ cells in human periphery can be expanded and acquire regulatory function when stimulated with allo-APC in the presence of IL-2 (Am. J. Transplant., supplement 11, vol. 5, p.257, 2005). This method is groundbreaking in that human regulatory T cells can be expanded and induced in vitro. However, because the CD45RA⁺CD25⁺CD4⁺ cells in periphery for use in the cultivation occur as a relatively small population of cells, it is necessary to draw a large amount of blood to increase the number of CD45RA⁺CD25⁺CD4⁺ cells at the start of cultivation, or to perform cultivation for a long period, when a large number of regulatory T cells are to be produced.

Meanwhile, Roncarolo et al. reported that when mouse CD4⁺T were stimulated with an antigen in the presence of rapamycin, CD4⁺CD25⁺FOXP3⁺ regulatory T cells were expanded selectively (Blood, vol. 105, No. 12, p.4743-4748, 2005). It is shown in the report that when isolated CD4⁺CD25⁺ T cells were stimulated in the presence of rapamycin, the cells proliferated vigorously and, as a result, produced a population of T cells having suppressive function, and comprising a high percentage of CD⁴⁺CD25^{bright+} T cells. Meanwhile, it is also shown that CD4⁺CD25⁻ T cells produced a population of T cells without suppressive function and did not enhance the expression of FOXP3 when stimulated in the presence of rapamycin. Therefore, according to the method of Roncarolo et al., CD4⁺CD25⁺ regulatory T cells can be expanded, but regulatory T cells cannot be produced from CD4⁺CD25⁻ T cells. Because the CD4⁺CD25⁺ cells in periphery used in the method occur a relatively small population, a large amount of blood must be drawn to prepare the cells.

Amid this situation, there has been a demand for the development of a method of efficiently producing regulatory T cells using a larger population of T cells that can easily be collected, and a compound that efficiently induces regulatory T cells.

Rapamycin is a macrocyclic triene antibiotic produced by Streptomyces hygroscopicus, and has been found to possess antifungal activity both in vitro and in vivo, particularly against Candida albicans [C. Vezina et al., J. Antibiot., 28, 721 (1975), S.N. Seghal et al., J. Antibiot., 28, 727(1975), H.A. Baker et al., J. Antibiot., 31, 539(1978), US Patent No. 3,929,992, and US Patent No. 3,993,749]. Furthermore, rapamycin has been shown to possess antitumor activity when used alone (US Patent No. 4,885,171) or in combination with picibanil (US Patent No. 4,401,653).

Immunosuppressive effects of rapamycin are disclosed in FASEB, 3, 3411(1989). It has also been shown that macrocyclic molecules such as cyclosporine A and FK-506 are effective as immunosuppressants, which are useful in the prevention of graft rejection [S.N. Sehgal, Transplant Proc., 35 (Suppl 3A), 7S-14S, 2003; R.Y. Calne et al., Lancet, 1183(1978); and US Patent No. 5,100,899]. Luo HY et al. disclosed that rapamycin was effective in an experimental allergic encephalomyelitis model (i.e., model of multiple sclerosis) and an adjuvant-induced arthritis model (i.e., model of rheumatoid arthritis), and effectively inhibited the formation of IgE-like antibody (Luo HY et al., Clin Immunol Immunopathol., 61(3):410-420, 1991).

Rapamycin has been shown to be effective in the prevention or treatment of systemic erythematosus [US Patent No. 5,078,899], lung inflammation [US Patent No. 5,080,899], insulin-dependent diabetes mellitus [US Patent No. 5,321,009], skin disorders, for example, psoriasis [US Patent No. 5,286,730], intestinal disorder [US Patent No. 5,286,731], smooth muscle cell proliferation and intimal hyperplasia after vascular injury [US Patent Nos. 5,288,711 and 5,516,781], adult T cell leukemia/lymphoma [European Patent Application 525,960 A1], eye inflammation [US Patent No. 5,387,589], malignant carcinoma [US Patent No. 5,206,018], inflammatory heart disease [US Patent No. 5,496,832] and anemia [US Patent No. 5,561,138].

In view of these circumstances, the present invention aims at providing a method of efficiently producing regulatory T cells using a larger population of T cells that are easy to be collected, and a screening method for a compound capable of efficiently inducing regulatory T cells.

### Disclosure of the Invention

The present inventors have conducted intensive studies in an attempt to achieve the above-mentioned object.

First, the present inventors attempted to culture CD25⁺CD4⁺ T cells having a naive phenotype for a long period to produce a large number of regulatory T cells. Thus, the present inventors found the problem of a gradual reduction in the immunosuppressive function of the acquired regulatory T cells during a long period of cultivation. To solve the problem, the present inventors conducted investigations of culture conditions, and found that the reduction in the immunosuppressive function can be prevented by culturing CD25⁺CD4⁺ T cells having a naive phenotype in the presence of a rapamycin compound. Furthermore, surprisingly, it was found that by using a rapamycin compound, regulatory T cells could be induced at high efficiency even from CD25⁻CD4⁺T cells having a naive phenotype, which had been thought to be unable to develop into regulatory T cells. Based on these and other findings, the present inventors found that by using a rapamycin compound, irrespective of the presence or absence of CD25 expression, regulatory T cells could be produced from CD4⁺T cells having a naive phenotype, and that by using the method of cultivation, a compound capable of efficiently inducing regulatory T cells could be screened for, and completed the present invention. Accordingly, the present invention relates to the following:
Accordingly, the present invention relates to the following:
   [1] A screening method for a compound capable of inducing regulatory T cells, comprising the following steps:
      (1) a step for culturing CD4⁺ T cells having at least one naive phenotype selected from the group consisting of (1) CD45RA⁺,
      (2) CD45RO^{low+} or CD45RO⁻, (3) CD45RO^{high+} and (4) CD38⁺ in the presence of a test compound, and isolating T cells from the culture product;
      (2) a step for evaluating the immunosuppressive function of the T cells isolated in the step (1);
      (3) a step for obtaining the foregoing test compound as a compound capable of inducing regulatory T cells if the evaluation in the step (2) shows that the T cells isolated in the step (1) have the immunosuppressive function.
   [2] The method described in [1], wherein the naive phenotype is CD45RA⁺.
   [3] The method described in [1], wherein the CD4⁺ T cells are CD25⁺.
   [4] The method described in [1], wherein the CD4⁺ T cells are CD25⁻.
   [5] The method described in [1], wherein the CD4⁺ T cells are of primate origin.
   [6] The method described in [1], wherein the CD4⁺ T cells are cultured in the presence of an antigen.
   [7] The method described in [1], wherein the CD4⁺ T cells are cultured in the presence of a T cell growth factor.
   [8] The method described in [1], wherein the test compound is an immunosuppressive compound or a derivative thereof.
   [9] A screening method for a compound capable of increasing the efficiency of production of regulatory T cells, comprising the following steps:
      (1) a step for culturing CD25⁺CD4⁺ T cells having at least one naive phenotype selected from the group consisting of (1) CD45RA⁺, (2) CD45RO^{low+} or CD45RO⁻, (3) CD45RO^{high+} and (4) CD38⁺ in the presence of a test compound to obtain regulatory T cells;
      (2) a step for evaluating the number and/or immunosuppressive function of the regulatory T cells obtained in the step (1);
      (3) a step for comparing the number and/or immunosuppressive function of the regulatory T cells evaluated in the step (2) with the number and/or immunosuppressive function of regulatory T cells obtained by culturing CD25⁺CD4⁺ T cells having at least one naive phenotype selected from the group consisting of (1) CD45RA⁺, (2) CD45RO^{low+} or CD45RO⁻, (3) CD45RO^{high+} and (4) CD38⁺ in the absence of the test compound;
      (4) a step for obtaining a test compound that has increased the number and/or immunosuppressive function of regulatory T cells as a compound capable of increasing the efficiency of production of regulatory T cells.
   [10] The method described in [9], wherein the naive phenotype in the steps (1) and (3) is CD45RA⁺.
   [11] The method described in [9], wherein the CD25⁺CD4⁺ T cells in the steps (1) and (3) are of primate origin.
   [12] The method described in [9], wherein the CD25⁺CD4⁺ T cells are cultured in the presence of an antigen in the steps (1) and (3).
   [13] The method described in [9], wherein the CD25⁺CD4⁺ T cells are cultured in the presence of a T cell proliferation factor in the steps (1) and (3).
   [14] The method described in [9], wherein the test compound is an immunosuppressive compound or a derivative thereof.
   [15] A method of producing regulatory T cells, comprising culturing CD4⁺ T cells having at least one naive phenotype selected from the group consisting of (1) CD45RA⁺, (2) CD45RO^{low+} or CD45RO⁻, (3) CD45RO^{high+}, and (4) CD38⁺ in the presence of a rapamycin compound to obtain regulatory T cells.
   [16] The method described in [15], wherein the naive phenotype is CD45RA⁺.
   [17] The method described in [15], wherein the CD4⁺ T cells are CD25⁺.
   [18] The method described in [15], wherein the CD4⁺ T cells are CD25⁻.
   [19] The method described in [15], wherein the CD4⁺ T cells are of primate origin.
   [20] The method described in [15], wherein the CD4⁺ T cells are cultured in the presence of an antigen.
   [21] The method described in [15], wherein the CD4⁺ T cells are cultured in the presence of a T cell growth factor.
   [22] The method described in [15], wherein the rapamycin compound is added to the medium 1 week after the start of cultivation or later.
   [23] The method described in [17], wherein the obtainable regulatory T cells have immunosuppressive function that depends on cell contact.
   [24] The method described in [18], wherein the obtainable regulatory T cells have immunosuppressive function that does not depend on cell contact.
   [25] The method described in [15], wherein the rapamycin compound is rapamycin.
   [26] The method described in [15], wherein the rapamycin compound is everolimus.
   [27] A method of maintaining the immunosuppressive function of regulatory T cells in producing regulatory T cells by culturing CD25⁺CD4⁺ T cells having at least one naive phenotype selected from the group consisting of (1) CD45RA⁺, (2) CD45RO^{low+} or CD45RO⁻, (3) CD45RO^{high+} and (4) CD38⁺ to obtain regulatory T cells, which comprise culturing the CD25⁺CD4⁺ T cells in the presence of a rapamycin compound.
   [28] A method of inducing regulatory T cells, comprising culturing CD25⁻CD4⁺ T cells having at least one naive phenotype selected from the group consisting of (1) CD45RA⁺, (2) CD45RO^{low+} or CD45RO⁻, (3) CD45RO^{high+} and (4) CD38⁺ in the presence of a rapamycin compound.
   [29] Regulatory T cells that can be obtained by the method according to [15], having at least one phenotype selected from one of the groups (A) to (C) below:
      (A) CD45RO^{mid+}, CD45RA^{mid+}, CD62L^{low+} and CD25^{high+}
      (B) CD45RO⁻, CD45RA^{mid+}, CD62L^{low+} and CD25^{high+}
      (C) CD45RO⁻, CD45RA^{high+}, CD62L^{high+} and CD25^{low+}.
   [30] Regulatory T cells having at least one phenotype selected from one of the groups (B) and (C) below:
      (B) CD45RO⁻, CD45RA^{mid+}, CD62L^{low+} and CD25^{high+}
      (C) CD45RO⁻, CD45RA^{high+}, CD62L^{high+} and CD25^{low+}.
   [31] An immunomodulator containing the regulatory T cells described in [29] or [30] as an active ingredient.
   [32] The agent described in [31], which is to be used for immunosuppression.
   [33] A method of producing an immunomodulator, comprising the following steps:
      (1) a step for culturing CD4⁺ T cells having at least one naive phenotype selected from the group consisting of (1) CD45RA⁺,
      (2) CD45RO^{low+} or CD45RO⁻, (3) CD45RO^{high+} and (4) CD38⁺ in the presence of a rapamycin compound to obtain regulatory T cells;
      (2) a step for mixing the regulatory T cells with a pharmaceutically acceptable carrier to give an immunomodulator.
   [34] The method described in [33], wherein the immunomodulator is to be used for immunosuppression.
   [35] An agent for inducing regulatory T cells from CD4⁺ T cells having at least one naive phenotype selected from the group consisting of (1) CD45RA⁺, (2) CD45RO^{low+} or CD45RO⁻, (3) CD45RO^{high+} and (4) CD38⁺, comprising a rapamycin compound.
   [36] The agent described in [35], wherein the CD4⁺ T cells are CD25⁻.
   [37] An agent for maintaining the immunosuppressive function of regulatory T cells in producing regulatory T cells by culturing CD4⁺ T cells having at least one naive phenotype selected from the group consisting of (1) CD45RA⁺, (2) CD45RO^{low+} or CD45RO⁻,
      (3) CD45RO^{high+} and (4) CD38⁺ to obtain regulatory T cells, comprising a rapamycin compound.
   [38] The agent described in [37], wherein the CD4⁺ T cells are CD25⁺.
   [39] A kit for production of regulatory T cells, comprising an antibody for preparing CD4⁺ T cells having at least one naive phenotype selected from the group consisting of (1) CD45RA⁺,
      (2) CD45RO^{low+} or CD45RO⁻, (3) CD45RO^{high+} and (4) CD38⁺, and a rapamycin compound.
   [40] A method of inducing CD25⁺CD4⁺ T cells having a naive phenotype in vivo, comprising the following steps:
      (1) a step for performing liver transplantation on a non-human mammal ;
      (2) a step for confirming the induction of CD25⁺CD4⁺ T cells having a naive phenotype in peripheral tissue of the foregoing non-human mammal.
   [41] A screening method for a compound capable of increasing the number of CD25⁺CD4⁺ T cells having a naive phenotype in vivo, comprising the following steps:
      (1) a step for performing liver transplantation on a non-human Mammal;
      (2) a step for administering a test compound to the mammal of the step (1);
      (3) a step for evaluating the number of CD25⁺CD4⁺ T cells having a naive phenotype in peripheral tissue of the mammal of the step (2);
      (4) a step for comparing the number of CD25⁺CD4⁺ T cells having a naive phenotype evaluated in the step (3) with the number of CD25⁺CD4⁺ T cells having a naive phenotype in peripheral tissue of a non-human mammal that has undergone liver transplantation, but has not received the test compound;
      (5) a step for selecting a compound that has increased the number of CD25⁺CD4⁺ T cells having a naive phenotype in the peripheral tissue.
   [42] A use of the regulatory T cells described in [29] or [30] for production of an immunomodulator.
   [43] A use of a rapamycin compound for producing an agent for inducing regulatory T cells from CD4⁺ T cells having at least one naive phenotype selected from the group consisting of (1) CD45RA⁺, (2) CD45RO^{low+} or CD45RO⁻, (3) CD45RO^{high+} and (4) CD38⁺.
   [44] A use of a rapamycin compound for producing an agent for maintaining the immunosuppressive function of regulatory T cells in producing regulatory T cells by culturing CD4⁺ T cells having at least one naive phenotype selected from the group consisting of (1) CD45RA⁺, (2) CD45RO^{low+} or CD45RO⁻, (3) CD45RO^{high+} and (4) CD38⁺ to obtain regulatory T cells.
   [45] A method of producing regulatory T cells, comprising culturing CD4⁺ T cells having a naive phenotype in the presence of a rapamycin compound to obtain regulatory T cells.
   [46] A method of producing an immunomodulator, comprising the following steps:
      (1) a step for culturing CD4⁺ T cells having a naive phenotype in the presence of a rapamycin compound to obtain regulatory T cells;
      (2) a step for mixing the regulatory T cells with a pharmaceutically acceptable carrier to produce an immunomodulator.
   [47] An agent for inducing regulatory T cells from CD4⁺ T cells having a naive phenotype, comprising a rapamycin compound.
   [48] An agent for maintaining the immunosuppressive function of regulatory T cells in producing regulatory T cells by culturing CD4⁺ T cells having a naive phenotype to obtain regulatory T cells, comprising a rapamycin compound.
   [49] A kit for production of regulatory T cells, comprising an antibody for preparing CD4⁺ T cells having a naive phenotype and a rapamycin compound.
   [50] A screening method for a compound capable of inducing regulatory T cells, comprising the following steps:
      (1) a step for culturing CD4⁺ T cells having a naive phenotype in the presence of a test compound, and isolating T cells from the culture product;
      (2) a step for evaluating the immunosuppressive function of the T cells isolated in the step (1);
      (3) a step for obtaining the foregoing test compound as a compound capable of inducing regulatory T cells if the evaluation in the step (2) shows that the T cells isolated in the step (1) have immunosuppressive function.
   [51] A screening method for a compound capable of increasing the efficiency of production of regulatory T cells, comprising the following steps:
      (1) a step for culturing CD25⁺CD4⁺ T cells having a naive phenotype in the presence of a test compound to obtain regulatory T cells;
      (2) a step for evaluating the number and/or immunosuppressive function of the regulatory T cells obtained in the step (1);
      (3) a step for comparing the number and/or immunosuppressive function of regulatory T cells evaluated in the step (2) with the number and/or immunosuppressive function of regulatory T cells obtained by culturing CD25⁺CD4⁺ T cells having a naive phenotype cultured in the absence of the test compound;
      (4) a step for obtaining a test compound that has increased the number and/or immunosuppressive function of regulatory T cells as a compound capable of increasing the efficiency of production of regulatory T cells.

By using the screening method of the present invention, a compound capable of inducing regulatory T cells can be obtained. The compound is useful as an immunosuppressive drug based on the new mechanism of inducing regulatory T cells, or as a seed for the development thereof.

Also, by using the method of the present invention, regulatory T cells can efficiently be produced from CD4⁺ T cells having a naive phenotype. CD4⁺ T cells having a naive phenotype occur as a relatively large population of T cells, and are easy to be prepared. The regulatory T cell produced by the method of the present invention can be used as an immunomodulator for the prophylaxis or treatment of the rejection in organ transplantation, an allergic disease, an autoimmune disease, a graft-versus-host disease (GVHD), infertility and the like.

### Brief Description of the Drawings

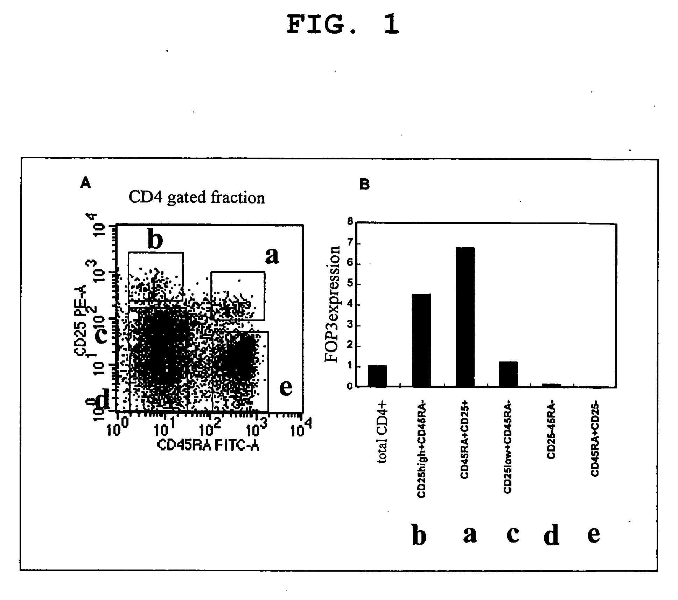

Fig. 1 shows the analysis results of CD45RA⁺CD25⁺CD4⁺ T cells and CD45RA⁺CD25⁻CD4⁺ T cells. (A) shows the definitions of (a) CD45RA⁺CD25⁺CD4⁺ T cell, (b) CD45RA⁻CD25^{high+}CD4⁺ T cell, (c) CD45RA⁻CD25^{low+}CD4⁺ T cell, (d) CD45RA⁻CD25⁻CD4⁺ T cell and (e) CD45RA⁺CD25⁻CD4⁺ T cell. (B) is a graph showing the FOXP3 expression level in each cell fraction.

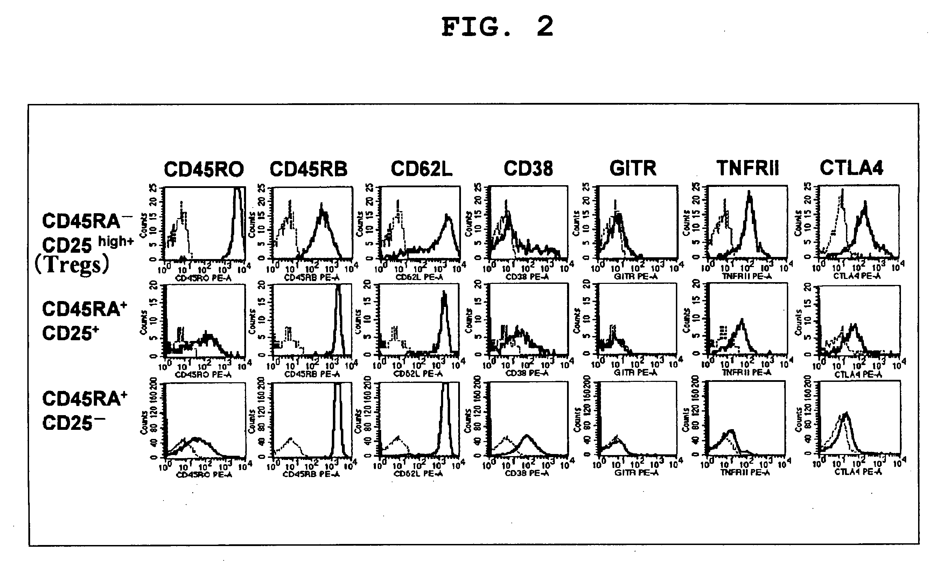

Fig. 2 shows the histogram analysis of naive/memory of CD45RA⁻CD25^{high+}CD4⁺, CD45RA⁺CD25⁺CD4⁺ and CD45RA⁺CD25⁻CD4⁺ cell fractions and the phenotype having a relatively high specificity to Treg. The analysis results using specific antibodies against the indicated antigens are shown with a solid line. The isotype control for each phenotype is shown with a dotted line.

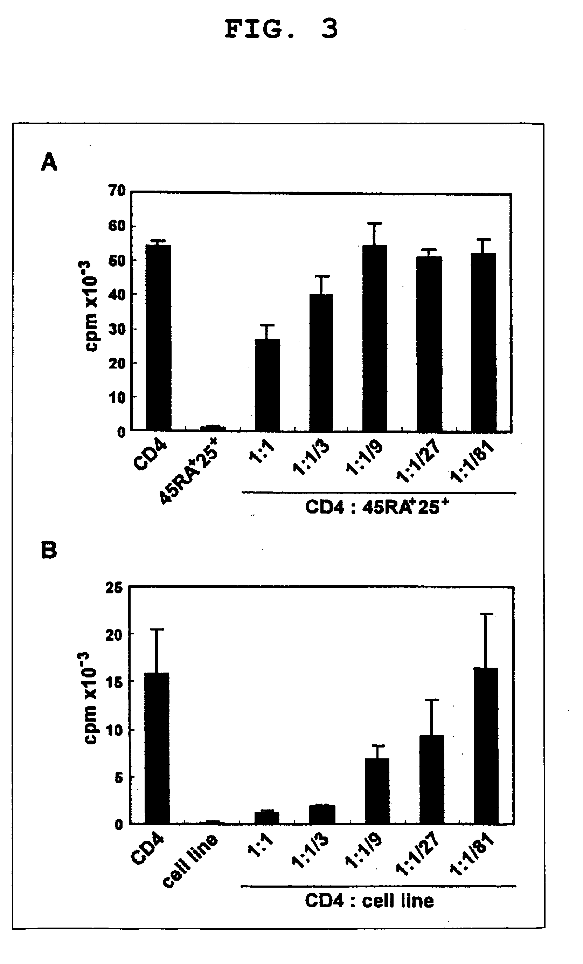

Fig. 3 shows the suppressive effect of (A) CD45RA⁺CD25⁺CD4⁺ T cell immediately after isolation and (B) CD45RA⁺CD25⁺CD4⁺ T cell lines (untreated with rapamycin) collected when the number of cells increased to about 100- to 150-fold, on the proliferation of total CD4⁺ T cells. The lanes of the bar graphs are referred to as lanes 1 - 7 from the left. Lane 1 (CD4): allogenic PBMC + total CD4⁺ T cells, lane 2 (45RA+25+): allogenic PBMC + CD45RA⁺CD25⁺CD4⁺ T cells, lanes 3 - 7: allogenic PBMC + total CD4⁺ T cells + CD45RA⁺CD25⁺CD4⁺ T cells (or T cell lines) (CD45RA⁺CD25⁺CD4⁺ T cells (or T cell lines) are added in the number of the indicated proportion (proportion relative to the number of total CD4⁺ T cells))o

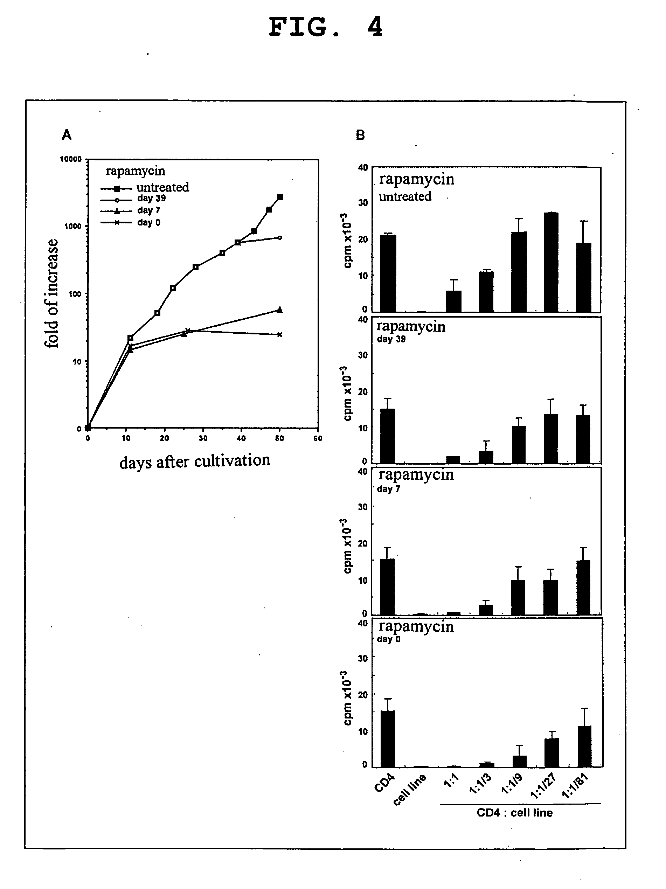

Fig. 4 shows the effects of rapamycin on the function of CD45RA⁺CD25⁺CD4⁺ T cell lines. (A) shows proliferation of CD45RA⁺CD25⁺CD4⁺ T cell line. The vertical axis shows the number of cells as a relative value when the number of cells at the start of the cultivation is 1, and the transverse axis shows the number of days after the start of the cultivation. Not treated: cultured in the absence of rapamycin. Day 0, 7 and 39: rapamycin was added at each number of days after the start of cultivation. (B) shows the suppressive effect of CD45RA⁺CD25⁺CD4⁺ T cell lines collected at 50 days from the start of cultivation on the proliferation of total CD4⁺ T cells. lane 1 (CD4): allogenic PBMC + total CD4⁺ T cells, lane 2 (cell line): allogenic PBMC + CD45RA⁺CD25⁺CD4⁺ T cell lines, lanes 3 - 7: allogenic PBMC + total CD4⁺ T cells + CD45RA⁺CD25⁺CD4⁺ T cell lines (CD45RA⁺CD25⁺CD4⁺ T cell lines are added in the number of the indicated proportion (proportion relative to the number of total CD4⁺ T cells)), Untreated: cultured in the absence of rapamycin. Day 0, 7 and 39: rapamycin was added at each number of days after the start of cultivation.

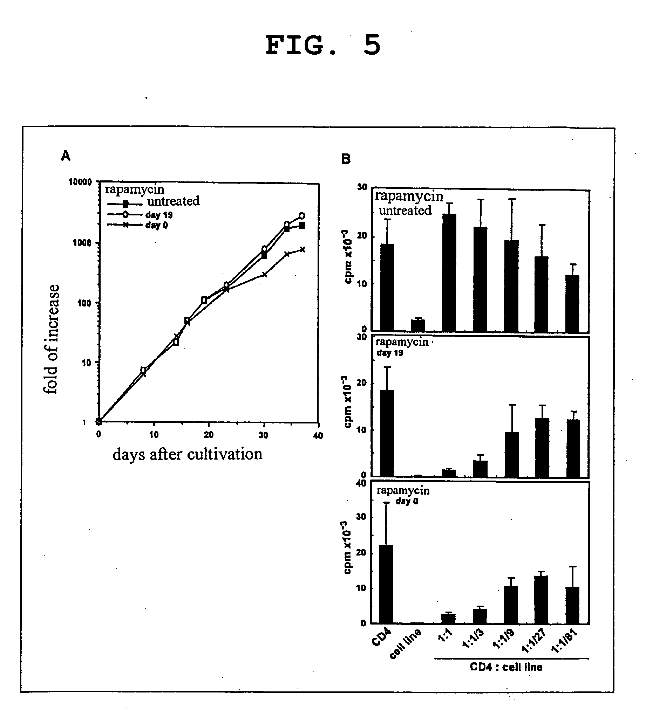

Fig. 5 shows the effects of rapamycin on the function of CD45RA⁺CD25⁻CD4⁺ T cell lines. (A) shows proliferation of CD45RA⁺CD25⁻CD4⁺ T cell lines. The vertical axis shows the number of cells as a relative value when the number of cells at the start of the cultivation is 1, and the transverse axis shows the number of days after the start of the cultivation. Not treated: cultured in the absence of rapamycin. Day 0 and 19: rapamycin was added at each number of days after the start of cultivation. (B) shows the effects of CD45RA⁺CD25⁻CD4⁺ T cell lines on the proliferation of total CD4⁺ T cells. Lane 1 (CD4): allogenic PBMC + total CD4⁺ T cell, lane 2 (cell line): allogenic PBMC + CD45RA⁺CD25⁻CD4⁺ T cell lines, lanes 3 - 7: allogenic PBMC + all CD4⁺ T cell + CD45RA⁺CD25⁻CD4⁺ T cell lines (CD45RA⁺CD25⁻CD4⁺ T cell lines are added in the number of the indicated proportion (proportion relative to the number of total CD4⁺ T cells)), Untreated: cultured in the absence of rapamycin. Day 0 and 19: rapamycin was added at each number of days after the start of cultivation.

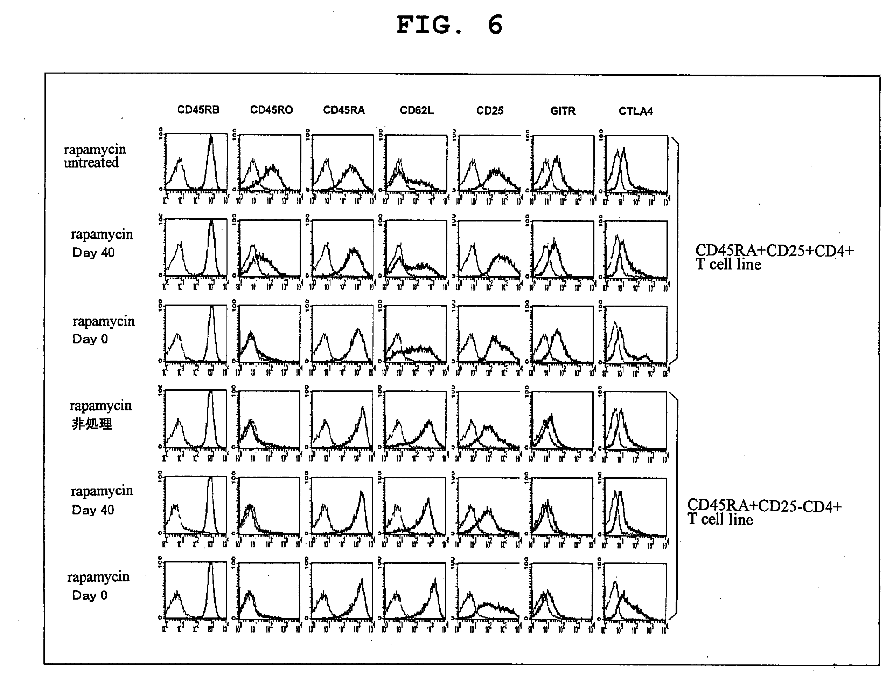

Fig. 6 shows the results of flow cytometric analysis showing differences in the phenotypes of CD45RA⁺CD25⁺CD4⁺ T cell lines and CD45RA⁺CD25⁻CD4⁺ T cell lines. The analysis results using specific antibodies against the indicated antigens are shown with a solid line. The isotype control for each phenotype is shown with a dotted line.

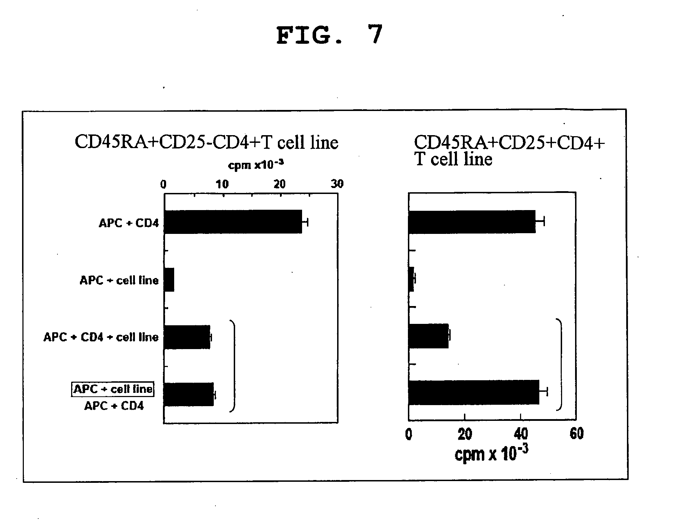

Fig. 7 shows cell contact dependency of the suppressive effect on the proliferation of total CD4⁺ T cells. The left panel shows the suppressive effect of CD45RA⁺CD25⁻CD4⁺ T cell lines, and the right panel shows the suppressive effect of CD45RA⁺CD25⁺CD4⁺ T cell lines. APC: allogenic PBMC, CD4: total CD4⁺ T cells, cell line: T cell line. The constituent elements enclosed with a rectangle were placed in the chamber above the trans-well.

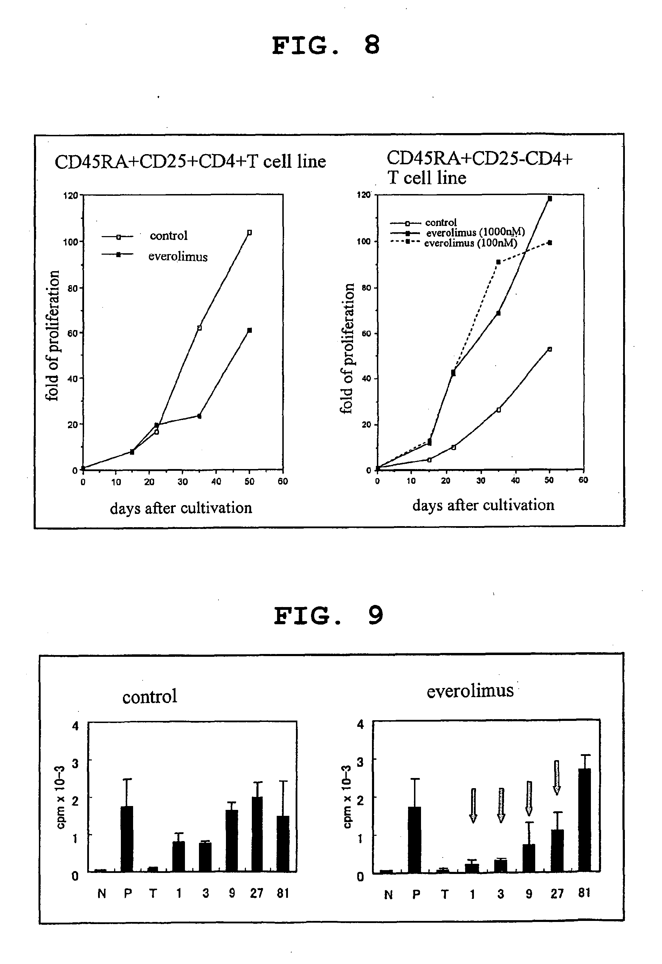

Fig. 8 shows the effects of everolimus on the proliferation of CD45RA⁺CD25⁺CD4⁺ T cell lines and CD45RA⁺CD25⁻CD4⁺ T cell lines. The vertical axis shows the number of cells as a relative value when the number of cells at the start of the cultivation is 1, and the transverse axis shows the number of days after the start of the cultivation.
Fig. 9 shows the effects of CD45RA⁺CD25⁺CD4⁺ T cell lines cultured in the presence or absence of everolimus on the proliferation of total CD4⁺ T cells. Lane 1 (N): total CD4⁺ T cells, lane 2 (P): allogenic PBMC + total CD4⁺ T cells, lane 3 (T): allogenic PBMC + CD45RA⁺CD25⁺CD4⁺ T cell lines, lanes 4 - 8: allogenic PBMC + total CD4⁺ T cells + CD45RA⁺CD25⁺CD4⁺ T cell lines (CD45RA⁺CD25⁺CD4⁺ T cell lines are added in the number of the indicated proportion (proportion relative to the number of total CD4⁺ T cells)).

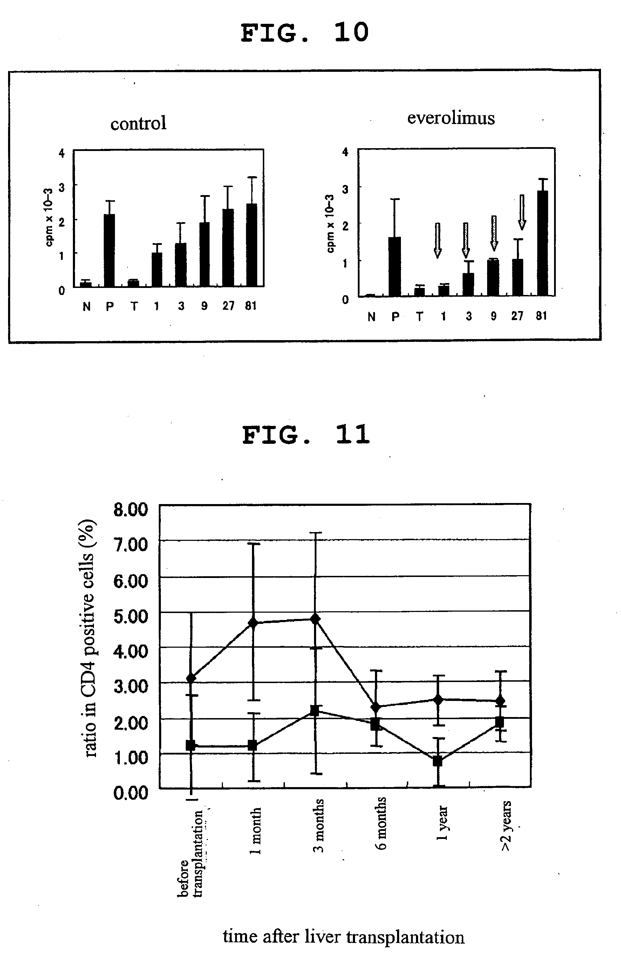

Fig. 10 shows the effects of CD45RA⁺CD25⁻CD4⁺ T cell lines cultivated in the presence or absence of everolimus on the proliferation of total CD4⁺ T cells. Lane 1 (N): total CD4⁺ T cells, lane 2 (P): allogenic PBMC + all CD4⁺ T cell, lane 3 (T): allogenic PBMC + CD45RA⁺CD25⁻CD4⁺ T cell lines, lanes 4 - 8: allogenic PBMC + total CD4⁺ T cell + CD45RA⁺CD25⁻CD4⁺ T cell lines (CD45RA⁺CD25⁻CD4⁺ T cell lines are added in the number of the indicated proportion (proportion relative to the number of total CD4⁺ T cell)).
Fig. 11 shows the shift in the rate of the number of CD45RA⁺CD25⁺CD4⁺ T cells in the peripheral blood after liver transplantation. ■: Children, ◆: Adults

### Detailed Description of the Invention

### (1. A method of producing a regulatory T cell)

The present invention provides a method of producing a regulatory T cell, which comprises culturing CD4⁺ T cell of a naive phenotype in the presence of a rapamycin compound to give the regulatory T cell.

The regulatory T cell means a T cell having an ability (immunosuppressive function) to inhibit activation (proliferation, production of cytokine etc.) of reactive T cells (e.g., total CD4⁺ T cell, CD25⁻CD4⁺ T cell etc.) when it is cultivated with the reactive T cell and subjected to T cell receptor-mediated stimulation. The regulatory T cells are generally present in a CD25⁺CD4⁺ T cell fraction having a memory phenotype (e.g., CD45RO⁺, CD45RA⁻, etc.) in the body. The regulatory T cell itself does not show a substantial proliferative reaction on T cell receptor-mediated stimulation, and can be unresponsive.

The CD4⁺ T cell having a naive phenotype to be used in the method of the present invention is generally derived from a mammal. While the mammal is not particularly limited as long as it can produce a regulatory T cell according to the method of the present invention, for example, experiment animals such as rodents (e.g., mouse, rat, hamster, guinea pig and the like), rabbit and the like; domestic animals such as swine, bovine, goat, horse, sheep, mink and the like; pets such as dog, cat and the like; and primates such as human, monkey, cynomolgus monkey, rhesus monkey, marmoset, orangutan, chimpanzee and the like can be mentioned. The mammal is preferably a primate, more preferably human.

The CD4⁺ T cells to be used in the method of the present invention can be prepared from any tissue, for example, a peripheral tissue (e.g., peripheral blood, spleen, lymph node, intestine, liver etc.), bone marrow, thymus, cord blood and the like. In consideration of easy preparation, the CD4⁺ T cells are preferably cells in peripheral blood or spleen.

The CD4⁺ T cell to be used for the method of the present invention is of a naive phenotype. The "naïve phenotype" refers to a phenotype of a T cells (naive T cells), which emigrate from thymus and are free of stimulation with an antigen. For example, the naive phenotype of human cell includes, but is not limited to, phenotypes such as CD45RA-positive (CD45RA⁺), CD45RO-low positive or CD45RO-negative (CD45RO^{low+} or CD45RO⁻), CD45RB-highly positive (CD45RB^{high+}), CD62L-highly positive (CD62L^{high+}), CD38-positive (CD38⁺) and the like. For example, in the case of human, the CD4⁺ T cells to be used for the method of the present invention can preferably be of at least one naive phenotype, more preferably all naïve phenotypes, selected from the group consisting of CD45RA⁺, CD45RO^{low+}, CD45RB^{high+}, CD62L^{high+} and CD38⁺. Even when the CD4⁺ T cells are derived from a mammal other than human, the phenotype may be the same as that of human. However, when the phenotype is defined by a marker molecule not inherently possessed by the animal species, interspecies difference can be taken into consideration, such as exclusion of the phenotype from the analysis and the like.

In the present specification, when the phenotype of a cell is represented by the presence or absence or the level of marker molecule (antigen) expression, unless otherwise specified, the phenotype is indicated by the presence or absence or the level of specific binding between an antibody and the marker molecule. The phenotype of a cell is generally determined by the presence or absence or the level of marker molecule expression by flow cytometric analysis and the like using an antibody specific to the marker molecule and the like. The "positive" expression of a marker molecule means that the marker molecule is expressed on the cell surface (or in the cell), and that a specific binding of an antibody against the marker molecule can be confirmed. Of these, the "highly positive" means, unless otherwise specified, that the level of expression of a marker molecule is relatively high, a cell population with a high level of expression of a marker molecule is present in a relatively large number, the rate of cell populations expressing a marker molecule is relatively large, and the like, as compared to other cells (or cell population) to be a control for comparison. The "low positive" means, unless otherwise specified, that the level of expression of a marker molecule is relatively low, cell populations with a low level of expression of a marker molecule is present in a relatively large number, the rate of cell populations expressing a marker molecule is relatively small, and the like, as compared to other cells (or cell population) to be a control for comparison.

When CD45RO-low positive (CD45RO^{low+}), CD45RB-highly positive (CD45RB^{high+}) or CD62L-highly positive (CD62L^{high+}) is indicated as a naïve phenotype, it means that memory T cell (T cell that emigrated from thymus, was subjected to stimulation with an antigen and still alive) is a control for comparison. As the memory T cell to be the control for comparison, CD45RA⁻CD25^{high+}CD4⁺ T cell (e.g., T cell contained in the region gated by b in Fig. 1A, etc.) and the like can be mentioned.

The CD4⁺ T cell of a naive phenotype to be used in the method of the present invention can be classified into CD25⁺ cell and CD25⁻ cell based on the presence or absence of CD25 expression CD25. The CD4⁺ T cell may be CD25⁺ cell, CD25⁻ cell or a mixture of CD25⁺ cell and CD25⁻ cell.

Of these, the CD25⁺CD4⁺ T cell of a naive phenotype can be of at least one phenotype selected from the group consisting of FOXP3-positive (FOXP3⁺), intracellular CTLA-4 positive (CTLA-4⁺) and TNFRII⁺. These phenotypes can be the phenotype of the aforementioned regulatory T cell (e.g., CD45RA⁻CD25^{high+}CD4⁺ T cell etc.). Of these, the CD25⁺CD4⁺ T cell of a naive phenotype may exhibit the intensity of FOXP3 expression of an equivalent (about 0.5 - 2.0 times) or above expression level as compared to that of CD45RA⁻CD25^{ligh+} CD4⁺ T cell, based on the expression level of mRNA. Phenotype analysis using FOXP3 is performed, generally by measuring the expression level of mRNA by RT-PCR and the like.

Meanwhile, the FOXP3 expression intensity in CD25⁻CD4⁺ T cells having a naive phenotype is extremely weak; the expression intensity can be normally about 1/4 or less, preferably about 1/20 or less, compared with regulatory T cells in periphery (e.g., CD45RA⁻CD25^{high+}CD4⁺ T cells and the like). CD25⁻CD4⁺ T cells having a naive phenotype, compared with regulatory T cells in periphery (e.g., CD45RA⁻CD25^{high+}CD4⁺ T cells and the like), exhibit extremely weaker expression of intracellular CTLA-4 and cell surface TNFRII; the expression level of intracellular CTLA-4 can be normally about 1/3 or less, preferably 1/8 or less, compared with CD45RA⁻CD25^{high+}CD4⁺ T cells, and the expression level of cell surface TNFRII can be normally about 1/3 or less, preferably 1/8 or less, compared with CD45RA⁻CD25^{high+}CD4⁺ T cells.

The CD4⁺ T cells of a naive phenotype to be used for the method of the present invention are preferably isolated or purified. The cells can be isolated and purified from the aforementioned mammalian tissue (peripheral tissue (e.g., peripheral blood, spleen, lymph node, intestine, liver etc.), bone marrow, thymus, cord blood, etc.) by a known method according to the above-mentioned phenotypes and the like.

For example, when a human CD4⁺ T cells of a naive phenotype are isolated and purified, a mononuclear cell fraction is prepared first from for example, peripheral blood, and the like. The mononuclear cell fraction is prepared, for example, by density gradient centrifugation using Ficoll-Hypaque (Amersham Biosciences-Uppsala) and the like, apheresis and the like. Then, the mononuclear cell fraction is stained with an antibody labeled with a fluorescence dye, magnetic beads or the like, which is specific to an antigen (CD4, the aforementioned naive phenotype marker antigen (CD45RA, CD45RB, CD62L, CD38 etc.) etc.) specifically expressed on the cell surface of the intended cell, and the intended fraction is isolated and purified using a cell sorter, a magnetic column or the like. The cell sorter is preferably used to achieve high purity.

Isolation and purification of CD25⁺ cells or CD25⁻ cells from CD4⁺T cells having a naive phenotype, if desired, can be performed as described above using a specific antibody against CD25 labeled with a fluorescent dye, magnetic beads and the like.

Next, the provided CD4⁺ T cells of a naive phenotype are cultured in the presence of a rapamycin compound.

For cell cultivation, the cultivation conditions generally used for lymphocyte cultivation technique can be used. For example, the cultivation temperature is generally within the range of about 30 - 40°C, preferably about 37°C. The CO₂ concentration is generally within the range of about 1 - 10%, preferably about 5%. The humidity is generally within the range of about 70 - 100%, preferably about 95 - 100%.

As the basal medium of a medium used for cultivation in the method of the present invention, a medium known per se can be used and is not particularly limited as long as a regulatory T cell can be produced by the method of the present invention. For example, DMEM, EMEM, RPMI-1640, α-MEM, F-12, F-10, M-199, HAM and the like can be mentioned. A modified medium (e.g., ALyS505N, etc.) for lymphocyte cultivation and the like can also be used, and a mixture of the above-mentioned basal media can also be used.

The medium can contain an additive known per se. While the additive is not particularly limited as long as a regulatory T cell can be produced by the method of the present invention, for example, an organic acid (e.g., sodium pyruvate etc.), an amino acid (e.g., L-glutamine etc.), a reducing agent (e.g., 2-mercaptoethanol etc.), a buffer (e.g., HEPES etc.), an antibiotic (e.g., streptomycin, penicillin, gentamicin etc.) and the like can be mentioned. Each of these additives is preferably contained within the concentration range known per se.

The medium may also contain a serum. The serum is not particularly limited as long as it is derived from mammal and a regulatory T cell can be produced by the method of the present invention. It is preferably a serum derived from the above-mentioned mammal (e.g., fetal bovine serum, human serum etc.). An alternative additive of serum (e.g., Knockout Serum Replacement (KSR) (manufactured by Invitrogen) etc.) can also be used. While the concentration of the serum is not particularly limited as long as a regulatory T cells can be produced by the method of the present invention, it is generally within the range of 0.1 - 30 (v/v) %.

To improve the production efficiency of a regulatory T cell according to the method of the present invention, a CD4⁺ T cells of a naive phenotype can be cultured in the presence of a T cell growth factor. While the T cell growth factor is not particularly limited as long as a regulatory T cell can be produced by the method of the present invention, for example, IL-2, IL-15, IFN-γ and the like can be mentioned. The concentration of the T cell growth factor to be added to the medium is not particularly limited as long as a regulatory T cell can be produced by the method of the present invention. For example, when IL-2 is used, the concentration is generally about 0.1 - 10000 U/ml, for example, 1 - 5000 U/ml, preferably 10 - 2500 U/ml.

As mentioned herein, "cultivation of CD4⁺ T cells having a naive phenotype in the presence of substance X" encompasses not only cases where CD4⁺ T cells having a naive phenotype are contained in the culture product when substance X is present, but also cases where CD4⁺ T cells having a naive phenotype are not contained in the culture product, but T cells derivatized from CD4⁺ T cells having a naive phenotype, and having a phenotype different from that of the CD4⁺ T cells having a naive phenotype, are contained, when substance X is present.

In the method of the present invention, a CD4⁺ T cell of a naive phenotype can be cultured in the presence of an antigen. A regulatory T cell specific to the antigen can be produced by cultivation in the presence of the antigen.

The antigen comprehensively means a substance that can be recognized by an antigen receptor (e.g., T cell receptor) on a cultured cell and can stimulate the cell via the receptor. The antigen includes, for example, not only an antigenic molecule such as peptide, protein, lipid, glycolipid and the like, but also immunologically non-self cell, an antigen mimic such as an agonistic antibody (e.g., OKT-3, which is anti-human CD3 antibody etc.), which recognizes a constituent molecule of an antigen receptor (CD3, TCRβ, TCRα etc.) or a costimulatory molecule (CD28 etc.), superantigen and the like. The immunologically non-self cell refers to a cell other than syngeneic cell, and allogenic cell and xenogenic cell can be mentioned. The immunologically non-self cell is preferably an allogenic cell.

As the antigen to be used for stimulation with an antigen, one desirable for the object can be selected.

For example, when a regulatory T cells specific to a particular immunologically non-autologous cell are to be produced, CD4⁺ T cells of a naive phenotype are cultured in the presence of rapamycin compound and the immunologically non-self cells. The immunologically non-self cells are preferably inactivated by a method known per se, for example, radiation (gamma-ray etc.), a treatment with an anticancer agent (mitomycin C etc.) and the like. The kind of the immunologically non-self cell is not particularly limited, and a cell derived from a desired tissue (e.g., peripheral blood mononuclear cell (PBMC) etc.) can be used. When a recipient-derived regulatory T cells specific to an allogenic donor-derived cell are to be produced before transplantation from the donor, for example, recipient-derived CD4⁺ T cells of a naive phenotype are cultured in the presence of rapamycin compound and the donor-derived cells. In this case, the donor-derived cells may be derived from the organ to be transplanted, or from a different tissue.

When a regulatory T cells specific to a particular antigen molecule (peptide, protein, lipid, glycolipid etc.) are to be produced, a CD4⁺ T cells of a naive phenotype are cultured in the presence of rapamycin compound and the antigen molecule. The antigen molecule includes; for example, cell- or tissue-derived antigen such as histocompatibility antigen and the like, a causative antigen of an allergic disease or a causative antigen of an autoimmune disease (a food-derived antigen, a pharmaceutical agent expected to exhibit antigenicity or a substance combined in a preparation, or an artificial organ-associated substance, or a denatured substance thereof (e.g., a thermally-denatured substance etc.)) and the like can be mentioned. As the histocompatibility antigen, major histocompatibility antigen (MHC antigen) and non-major histocompatibility antigen can be mentioned. The causative substance of allergy includes environmental or pollen antigen, fungal antigen, food antigen, artificial antigen and the like. For example, as the environmental or pollen antigen, mite, house dust, Japanese cedar pollen, ragweed and the like can be mentioned. As the fungal antigen, Candida, Alternaria, Aspergillus, cladosporium, Penicillium and the like can be mentioned. As the food antigen, albumen, milk, soybean, flour, buckwheat flour, mackerel, sardine, Japanese horse mackerel, shrimp, crab, pork, beef, chicken and the like can be mentioned. As the artificial antigen, a pharmaceutical agent, an artificial organ and the like can be mentioned. As the causative substance of autoimmune disease, a corresponding antigen to an autoantibody causing the disease, and the like can be mentioned.

In this case, the CD4⁺ T cells may be cultured in the presence of an antigen-presenting cell in order to certainly accomplish antigen presentation to the cell. While the antigen-presenting cell is not particularly limited as long as regulatory T cells can be produced by the method of the present invention, a antigen-presenting cell syngeneic to the CD4⁺ T cells of a naive phenotype to be cultivated (e.g., antigen-presenting cell obtained from the individual from which the CD4⁺ T cells are derived) is generally used. While the kind of the antigen-presenting cell is not particularly limited as long as it has an antigen-presenting ability and can produce regulatory T cells by the method of the present invention, for example, PBMC, dendritic cell and the like can be used. The antigen-presenting cell is preferably inactivated by a method known per se, for example, radiation (gamma-ray etc.), a treatment with an anticancer agent (mitomycin C etc.) and the like.

In addition, when the repertoire variety of the antigen receptors possessed by the above-mentioned CD4⁺ T cell population to be cultivated is to be maintained to produce a regulatory T cell population reflecting the variety, the CD4⁺ T cells are cultured under a stimulation with an antigen mimic such as an agonistic antibody (e.g., OKT-3, which is an anti-human CD3 antibody etc.), which recognizes a constituent molecule of an antigen receptor (CD3, TCRβ, TCRα etc.), an agonistic antibody (e.g., anti-human CD28 antibody), which recognizes a costimulatory molecule (CD28 etc.), a superantigen and the like (Blood, 104, p. 895-903, 2004, Blood, 104, p. 453-61, 2004). Plural kinds of the antigen mimic can be used in combination and, for example, a combination of an agonistic antibody recognizing CD3 and an agonistic antibody recognizing CD28 and the like can be used. Using the antigen mimic, regulatory T cell populations having variety can be produced.

In addition, plural kinds of antigens can also be used in combination and, for example, a combination of an immunologically non-self cell and an agonistic antibody recognizing a constituent molecule of an antigen receptor (combination of allogenic cell and anti-CD3 antibody etc.) can be used.

In the method of the present invention, CD4⁺ T cells having a naive phenotype are cultured in the presence of a rapamycin compound. By using a rapamycin compound, immunosuppressive function is induced in CD4⁺ T cells having a naive phenotype or T cells derived from the cells (sometimes referred to as a CD4⁺ T cell line), the reduction in the immunosuppressive function of regulatory T cells that can accompany cell proliferation at high ratios is suppressed, and the immunosuppressive function of regulatory T cells is maintained at high levels, or the immunosuppressive function of regulatory T cells is enhanced.

Here, while regulatory T cells can also be obtained by culturing CD25⁺CD4⁺ T cells having a naive phenotype in the absence of a rapamycin compound (Am. J. Transplant., supplement 11, vol.5, p.257, 2005), by using a rapamycin compound, it is possible to particularly suppress the reduction in the immunosuppressive function of regulatory T cells that can accompany cell proliferation at high ratios, to maintain the immunosuppressive function of regulatory T cells at high levels, or to enhance the immunosuppressive function of regulatory T cells.

Meanwhile, although it is difficult to obtain regulatory T cells if CD25⁻CD4⁺ T cells having a naive phenotype are cultured in the absence of a rapamycin compound, by using a rapamycin compound, immunosuppressive function is induced in CD25⁻CD4⁺ T cells having a naive phenotype or T cells derived from the cells, the immunosuppressive function of the induced regulatory T cells is maintained at high levels, or the immunosuppressive function of regulatory T cells is enhanced, with the use of a rapamycin compound.

As used herein, the term "rapamycin compound" defines a class of immunosuppressive compound comprising the basic rapamycin nucleus (shown below) (also referred to as rapamycins). Rapamycin compounds include compounds that may be chemically or biologically modified as derivatives of the rapamycin nucleus while retaining an immunosuppressive property. Therefore, the term "rapamycin compound" encompasses esters, ethers, oximes, hydrazones and hydroxylamines of rapamycin, and rapamycin compounds wherein a functional group on the rapamycin nucleus is modified by, for example, reduction or oxidation. The term "rapamycin compound" encompasses pharmaceutically acceptable salts of rapamycin compounds. Rapamycin compounds are capable of forming a salt by containing an acidic or basic moiety.

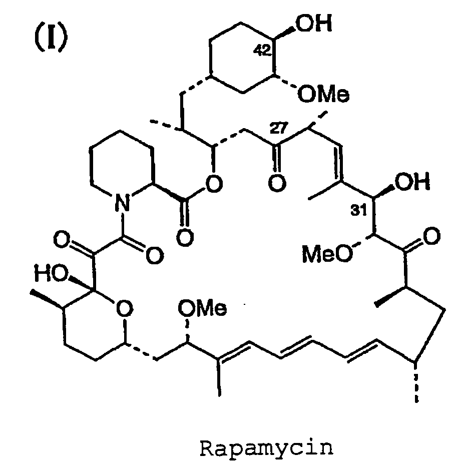

The esters and ethers of rapamycin are preferably those with respect to the hydroxyl group at the 42-position and/or 31-position of the rapamycin nucleus, or esters and ethers of the hydroxyl group at the 27-position (after chemical reduction of 27-ketone); the oximes, hydrazones and hydroxylamines of rapamycin are preferably those with respect to the ketone at the 42-position (after oxidation of the 42-hydroxyl group), or those with respect to the 27-ketone of the rapamycin nucleus.

Preferable 42- and/or 31-esters and ethers of rapamycin are disclosed in the following patents (all disclosures therein are deemed to be herein incorporated by reference): alkyl ester (US Patent No. 4,316,885); aminoalkyl ester (US Patent No. 4,650,803); fluorinated ester (US Patent No. 5,100,883); amide ester (US Patent No. 5,118,677); carbamate ester (US Patent No. 5,118,678); silyl ether (US Patent No. 5,120,842); amino ester (US Patent No. 5,130,307); acetal (US Patent No. 5,151,413); amino diester (US Patent No. 5,162,333); sulfonate and sulfate esters (US Patent No. 5,177,203); ester (US Patent No. 5,221,670); alkoxy ester (US Patent No. 5,233,036); O-aryl, -alkyl, -alkenyl and -alkynyl ethers (US Patent No. 5,258,389); carbonate ester (US Patent No. 5,260,300); arylcarbonyl and alkoxycarbonyl carbamates (US Patent No. 5,262,423); carbamate (US Patent No. 5,302,584); hydroxy ester (US Patent No. 5,362,718); hindered ester (US Patent No. 5,385,908); heterocyclic ester (US Patent No. 5,385,909); gem-disubstituted ester (US Patent No. 5,385,910); aminoalkane ester (US Patent No. 5,389,639); phosphorylcarbamate ester (US Patent No. 5,391,730); carbamate ester (US Patent No. 5,411,967); carbamate ester (US Patent No. 5,434,260); amidinocarbamate ester (US Patent No. 5,463,048); carbamate ester (US Patent No. 5,480,988); carbamate ester (US Patent No. 5,480,989); carbamate ester (US Patent No. 5,489,680); hindered N-oxide ester (US Patent No. 5,491,231); biotin ester (US Patent No. 5,504,091); O-alkyl ether (US Patent No. 5,665,772); and PEG ester of rapamycin (US Patent No. 5,780,462). Production of these esters and ethers is disclosed in the aforementioned patents.

Disclosed in JP A-8-502266 (US Patent No. 5,665,772) is a suitable rapamycin compound represented by the formula (II): 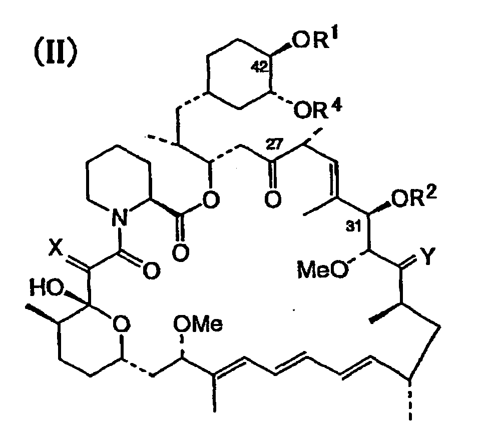 [wherein X is (H,H) or O;
Y is (H,OH) or O;
R¹ and R² are independently selected from the group consisting of H, alkyl, thioalkyl, arylalkyl, hydroxyalkyl, dihydroxyalkyl, hydroxyalkylarylalkyl, dihydroxyalkylarylalkyl, alkoxyalkyl, acyloxyalkyl, aminoalkyl, alkylaminoalkyl, alkoxycarbonylaminoalkyl, acylaminoalkyl, arylsulfonamidalkyl, allyl, dihydroxyalkylallyl, dioxolanylallyl, carboalkoxyalkyl and (R³)₃Si (wherein, each R³ is independently selected from among H, methyl, ethyl, isopropyl, t-butyl and phenyl); here, the term "alk-" or "alkyl" means a branched or linear C₁₋₆ alkyl, preferably a C₁₋₃ alkyl, whose carbon chain may be interrupted by an ether (-O-) linkage as desired; and R⁴ represents methyl or R⁴ and R¹ bind together to form a C₂₋₆ alkylene;
wherein neither R¹ nor R² is H; and if R¹ is (R³)₃Si or carboalkoxyalkyl, neither X nor Y is O.]

The compound of the formula (II) is preferably a 42-O-substituted rapamycin wherein X and Y are both O, R² is H, R⁴ is methyl and R¹ is not H; most preferably, R¹ is selected from among hydroxyalkyl, hydroxy alkoxyalkyl, acylaminoalkyl and aminoalkyl; particularly 42-O-(2-hydroxy)ethyl-rapamycin, 42-O-(3-hydroxy)propyl-rapamycin, 42-O-[2-(2-hydroxy)ethoxy]ethyl-rapamycin and 42-O-(2-acetaminoethyl)-rapamycin are preferable.

US Patent No. 5,258,389 discloses suitable rapamycin compounds represented by the following formula (III), or pharmaceutically acceptable salts thereof. 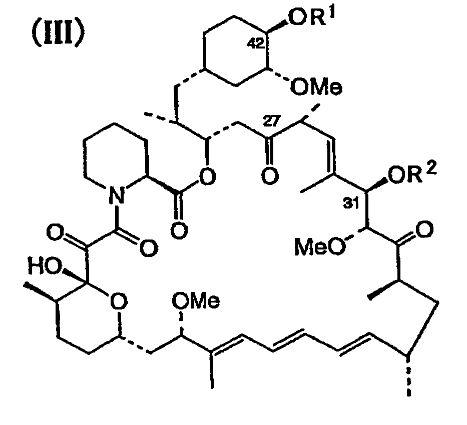 [wherein R¹ and R² are independently selected from among the following:
(1) hydrogen;
(2) phenyl;
(3) substituted phenyl wherein the substituents are X, Y and Z;
(4) 1- or 2-naphthyl;
(5) substituted 1- or 2-naphthyl wherein the substituents are X, Y and Z;
(6) biphenyl;
(7) substituted biphenyl wherein the substituents are X, Y and Z;
(8) C₁₋₁₀ alkyl;
(9) substituted C₁₋₁₀ alkyl wherein one or more substituents are selected from among the following:
   (a) hydroxy,
   (b) oxo,
   (c) C₁₋₆ alkoxy,
   (d) phenyl-C₁₋₃ alkoxy,
   (e) substituted phenyl-C₁₋₃ alkoxy wherein the substituents on the phenyl are X, Y and Z,
   (f) -OCO-C₁₋₆ alkyl,
   (g) -NR⁶R⁷, here R⁶ and R⁷ are independently selected from among the following:
      (i) hydrogen,
      (ii) C₁₋₁₀ alkyl not substituted or substituted by one or more substituents selected from among the following:
         (a') phenyl not substituted or substituted by X, Y and Z,
         (b') -OH,
         (c') C₁₋₆ alkoxy,
         (d') -CO₂H,
         (e') -CO₂-C₁₋₆ alkyl,
         (f') -C₃₋₇ cycloalkyl, and
         (g') -OR¹¹,
      (iii) C₃₋₁₀ alkenyl not substituted or substituted by one or more substituents selected from among the following:
         (a') phenyl not substituted or substituted by X, Y and Z,
         (b') -OH,
         (c') C₁₋₆ alkoxy,
         (d') -CO₂H,
         (e') -CO₂-C₁₋₆ alkyl,
         (f') -C₃₋₇ cycloalkyl, and
         (g') -OR¹¹,
      (iv) or R⁶ and R⁷ and N having the same attaching thereto are capable of forming a 3-7-membered saturated heterocyclic ring not substituted or substituted by a C₁₋₆ alkyl or phenyl, the ring being selected from the group consisting of aziridine, morpholine, thiomorpholine, thiomorpholine-oxide, thiomorpholine-dioxide, piperidine, pyrrolidine, and piperidine,
   (h) -NR⁶CO-C₁₋₆ alkyl-R⁷, here R⁶ is as defined above,
   (i) -NR⁶CO₂-C₁₋₆ alkyl-R⁷,
   (j) -NR⁶CONR⁶R⁷,
   (k) -OCONR⁶R⁷,
   (l) -COOR⁶,
   (m) -CHO,
   (n) phenyl,
   (o) substituted phenyl wherein the substituents are X, Y and Z,
   (p) phenyloxy,
   (q) substituted phenyloxy wherein the substituents are X, Y and Z,
   (r) 1- or 2-naphthyl,
   (s) substituted 1- or 2-naphthyl wherein the substituents are X, Y and Z,
   (t) biphenyl,
   (u) substituted biphenyl wherein the substituents are X, Y and Z,
   (v) -OR¹¹, and
   (w) -S(O) p-C₁₋₆ alkyl;
(10) C₃₋₁₀ alkenyl;
(11) substituted C₃₋₁₀ alkenyl wherein one or more substituents being selected from among the following:
   (a) hydroxy,
   (b) oxo,
   (c) C₁₋₆ alkoxy,
   (d) phenyl-C₁₋₃ alkoxy,
   (e) substituted phenyl-C₁₋₃ alkoxy wherein the substituent on the phenyl is X, Y and Z,
   (f) -OCO-C₁₋₆ alkyl,
   (g) -NR⁶R⁷, here R⁶ and R⁷ are as defined above,
   (h) -NR⁶CO-C₁₋₆ alkyl wherein R⁶ is as defined above,
   (i) -COOR⁶ wherein R⁶ is as defined above,
   (j) -CHO,
   (k) phenyl,
   (l) substituted phenyl wherein the substituents are X, Y and Z,
   (m) 1- or 2-naphthyl,
   (n) substituted 1- or 2-naphthyl wherein the substituents are X, Y and Z,
   (o) biphenyl,
   (p) substituted biphenyl wherein the substituents are X, Y and Z,
   (q) -OR¹¹, and
   (r) -S(O) ₚ-C₁₋₆ alkyl;
(12) C₃₋₁₀ alkynyl;
(13) substituted C₃₋₁₀ alkynyl wherein one or more substituents are selected from among the following:
   (a) hydroxy,
   (b) oxo,
   (c) C₁₋₆ alkoxy,
   (d) phenyl-C₁₋₃ alkoxy,
   (e) substituted phenyl-C₁₋₃ alkoxy wherein the substituent on the phenyl is X, Y and Z,
   (f) -OCO-C₁₋₆ alkyl,
   (g) -NR⁶R⁷, here R⁶ and R⁷ are as defined above,
   (h) -NR⁶CO-C₁₋₆ alkyl, here R⁶ is as defined above,
   (i) -COOR⁶, here R⁶ is as defined above,
   (j) -CHO,
   (k) phenyl,
   (l) substituted phenyl wherein the substituent are X, Y and Z,
   (m) 1- or 2-naphthyl,
   (n) substituted 1- or 2-naphthyl wherein the substituents are X, Y and Z,
   (o) biphenyl,
   (p) substituted biphenyl wherein the substituents are X, Y and Z, and
   (q) -OR¹¹-;
wherein R¹ and R² are not concurrently hydrogen;
R¹¹ is selected from among the following:
(a) -PO(OH) O⁻M⁺, here M⁺ is a positively charged inorganic or organic counterion,
(b) -SO₃⁻M⁺,
(c) -CO(CH₂)_{q}CO₂⁻M⁺, here q is 1-3, and
(d) -CO-C₁₋₆ alkyl-NR⁶R⁷, here R⁶ and R⁷ are as defined above, and the alkyl is not substituted or substituted by one or more substituents selected from among the following:
   (i) hydroxy,
   (ii) C₁₋₆ alkoxy,
   (iii) -NR¹⁶R¹⁷, here R¹⁶ and R¹⁷ are independently selected from among the following:
      (a') hydrogen, and
      (b') C₁₋₆ alkyl,
   (iv) -COOR⁶, here R⁶ is as defined above,
   (v) phenyl,
   (vi) substituted phenyl wherein the substituents are X, Y and Z,
   (vii) -SH, and
   (viii) -S-C₁₋₆ alkyl;
X, Y and X are independently selected from among the following:
(a) hydrogen,
(b) C₁₋₇ alkyl,
(c) C₂₋₆ alkenyl,
(d) halogen,
(e) - (CH₂) ₘ-NR⁶R⁷, here R⁶ and R⁷ are as defined above, and m is 0 to 2,
(f) -CN,
(g) -CHO,
(h) -CF₃,
(i) -SR⁸, here R⁸ is hydrogen, C₁₋₆ alkyl, trifluoromethyl, or phenyl,
(j) -SOR⁸, here R⁸ is as defined above,
(k) -SO₂R⁸, here R⁸ is as defined above,
(l) -CONR⁶R⁷, here R⁶ and R⁷ are as defined above,
(m) R⁹O(CH₂) ₘ-, here R⁹ is hydrogen, C₁₋₃ alkyl, hydroxy-C₂₋₃ alkyl, trifluoromethyl, phenyl or naphthyl, and m is as defined above,
(n) -CH(OR¹²) (OR¹³), here R¹² and R¹³ are C₁₋₃ alkyl, or bind together to form an ethyl or propyl bridge,
(o) 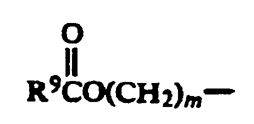 here R⁹ and m are as defined above, and
(p) 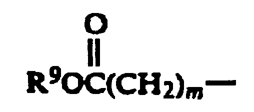
(q) -OR¹¹;
or any two adjoining members of X, Y and Z may bind together to form a ring selected from the group consisting of dioxolanyl, dihydrofuranyl, dihydropyranyl, and dioxanyl.]

Disclosed in JP A-9-512018 (US Patent No. 5,362,718) are suitable rapamycin compounds represented by the following formula (IV), or pharmaceutically acceptable salts thereof. 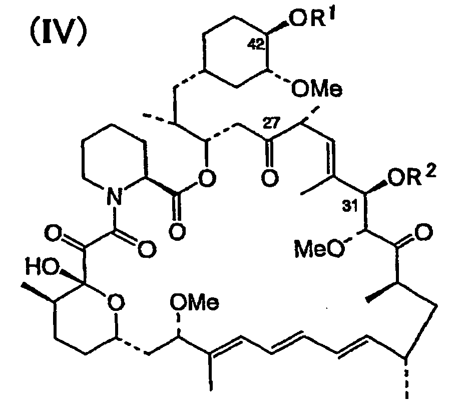 [wherein R¹ and R² are independently hydrogen or -CO (CR³R⁴) _{b} (CR⁵R⁶) _{d}CR⁷R⁸R⁹ ;
R³ and R⁴ are independently hydrogen, an alkyl having 1 to 6 carbon atoms, an alkenyl having 2 to 7 carbon atoms, an alkynyl having 2 to 7 carbon atoms, trifluoromethyl, or -F;
R⁵ and R⁶ are independently hydrogen, an alkyl having 1 to 6 carbon atoms, an alkenyl having 2 to 7 carbon atoms, an alkynyl having 2 to 7 carbon atoms, -(CR³R⁴)_{f}OR¹⁰, -CF₃, -F, or -CO₂R¹¹, or R⁵ and R⁶ may bind together to form X or a cycloalkyl ring having 3 to 8 carbon atoms (as desired, mono-, di-, or trisubstituted by -(CR³R⁴)_{f}OR¹⁰) ;
R⁷ is hydrogen, an alkyl having 1 to 6 carbon atoms, an alkenyl having 2 to 7 carbon atoms, an alkynyl having 2 to 7 carbon atoms, -(CR³R⁴)_{f}OR¹⁰, -CF³, -F, or -CO₂R¹¹;
R⁸ and R⁹ are independently hydrogen, an alkyl having 1 to 6 carbon atoms, an alkenyl having 2 to 7 carbon atoms, an alkynyl having 2 to 7 carbon atoms, -(CR³R⁴)_{f}OR¹⁰, -CF₃, -F, or -CO₂R¹¹, or R⁸ and R⁹ may bind together to form X or a cycloalkyl ring having 3 to 8 carbon atoms (as desired, mono-, di-, or trisubstituted by -(CR³R⁴)_{f}OR¹⁰);
R¹⁰ is hydrogen, an alkyl having 1 to 6 carbon atoms, an alkenyl having 2 to 7 carbon atoms, an alkynyl having 2 to 7 carbon atoms, a tri-(alkyl having 1 to 6 carbon atoms)silyl, a tri-(alkyl having 1 to 6 carbon atoms)silylethyl, triphenylmethyl, benzyl, an alkoxymethyl having 2 to 7 carbon atoms, a tri-(alkyl having 1 to 6 carbon atoms)silylethoxymethyl, chloroethyl, or tetrahydropyranyl;
R¹¹ is hydrogen, an alkyl having 1 to 6 carbon atoms, an alkenyl having 2 to 7 carbon atoms, an alkynyl having 2 to 7 carbon atoms, or a phenylalkyl having 7 to 10 carbon atoms;
X is a 5-(2,2-di-(alkyl having 1 to 6 carbon atoms))[1,3] dioxanyl, a 5-(2-spiro(cycloalkyl having 3 to 8 carbon atoms))[1,3] dioxanyl, a 4-(2,2-di-(alkyl having 1 to 6 carbon atoms))[1,3] dioxanyl, a 4-(2-spiro(cycloalkyl having 3 to 8 carbon atoms))[1,3] dioxanyl, a 4-(2,2-di-(alkyl having 1 to 6 carbon atoms))[1,3] dioxalanyl, or a 4-(2-spiro(cycloalkyl having 3 to 8 carbon atoms))[1,3] dioxalanyl;
b=0 to 6;
d=0 to 6; and f=0 to 6; however, R¹ and R² are not concurrently hydrogen,
furthermore, either R¹ or R² contains at least one -(CR³R⁴)_{f}OR¹⁰, X, or cycloalkyl group having 3 to 8 carbon atoms group substituted by -(CR³R⁴)_{f}OR¹⁰.]

In the formula (IV), the terms alkyl having 1 to 6 carbon atoms, alkenyl having 2 to 7 carbon atoms, and alkynyl having 2 to 7 carbon atoms are understood to encompass both linear and branched carbon chains. Because the compounds of the formula (IV) contain one or more -(CR³R⁴)_{f}OR¹⁰ groups, R³, R⁹, f and R¹⁰ can be identical or different. Likewise, if another comprehensive explanation concerning substituents is repeated for a structure, the substituents can be identical or different. In the case of a compound wherein R¹ comprises R⁸ and R⁹ that bind together to form X [here, X is a 5-(2,2-di-(alkyl having 1 to 6 carbon atoms))[1,3] dioxanyl], the alkyl group for X contains one carbon atom, and d=0, then R¹ has the following structure:

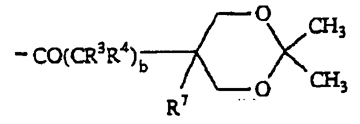

Likewise, in the case of a compound wherein R¹ comprises R⁸ and R⁹ that bind together to form X [here, X is a 4-(2-spiro(cycloalkyl having 3 to 8 carbon atoms))[1,3] dioxanyl], the cycloalkyl group for X has six carbon atoms, and d=0, then R¹ has the following structure:

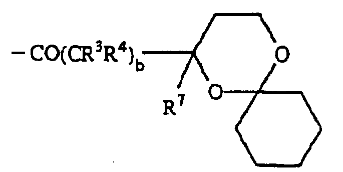

Of the X-containing compounds of the formula (IV), a preferable compound is one wherein the alkyl group for X, if any, is methyl, and the cycloalkyl group for X, if any, is cyclohexyl.

Provided that R¹⁰ is not hydrogen, alkyl, alkenyl, or alkynyl, this means that R¹⁰ is a group capable of functioning as an alcohol-protecting group. Hence, these groups are essentially not only biologically active, but also intermediates for free hydroxylated compounds. R¹⁰ includes tri-(alkyl having 1 to 6 carbon atoms)silyl, tri-(alkyl having 1 to 6 carbon atoms)silylethyl, triphenylmethyl, benzyl, alkoxymethyl having 2 to 7 carbon atoms, tri-(alkyl having 1 to 6 carbon atoms)silylethoxymethyl, chloroethyl, and tetrahydropyranyl groups. Other alcohol-protecting groups are known to those skilled in the art, and can also be deemed parts of compounds of the formula (IV).

Of the compounds of the formula (IV), preferable members are one wherein R² is hydrogen; one wherein R² is hydrogen, b=0, and d=0; and one wherein R² is hydrogen, b=0, d=0, and R⁸ and R⁹ are independently hydrogen, alkyl, or -(CR³R⁴)_{f}OR¹⁰, or R⁸ and R⁹ bind together to form X. Of the compounds of the formula (IV), a particularly preferable member is the rapamycin 42-ester with 3-hydroxy-2-(hydroxymethyl)-2-methylpropionic acid.

Accordingly, examples of rapamycin compounds include compounds disclosed in any one of the aforementioned patents, represented by the formula: 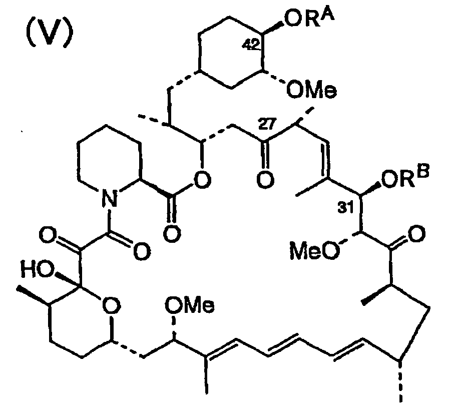 [wherein R^{A} and R^{B} are selected from among hydrogen and ester- or ether-forming groups].

Preferable 27-esters and ethers of rapamycin are disclosed in US Patent No. 5,256,790 (all disclosures therein are deemed to be herein incorporated by reference). Production of these esters and ethers is disclosed in the aforementioned patents.

Preferable oximes, hydrazones and hydroxylamines of rapamycin are disclosed in US Patent Nos. 5,373,014, 5,378,836, 5,023,264 and 5,563,145 (all disclosures therein are deemed to be herein incorporated by reference). Production of these oximes, hydrazones and hydroxylamines is disclosed in the aforementioned patents. Production of 42-oxorapamycin is disclosed in US Patent No. 5,023,263 (all disclosures therein are deemed to be herein incorporated by reference).

Particularly preferable rapamycin compounds include rapamycin [US Patent No. 3,929,992], the rapamycin 42-ester with 3-hydroxy-2-(hydroxymethyl)-2-methylpropionic acid [US Patent No. 5,362,718] and 42-O-(2-hydroxy)ethyl-rapamycin [US Patent No. 5,665,772] (everolimus).

Whenever applicable, pharmaceutically acceptable salts can be formed from organic acids and inorganic acids, for example, acetic acid, propionic acid, lactic acid, citric acid, tartaric acid, succinic acid, fumaric acid, maleic acid, malonic acid, mandelic acid, malic acid, phthalic acid, hydrochloric acid, hydrobromic acid, phosphoric acid, nitric acid, sulfuric acid, methanesulfonic acid, naphthalenesulfonic acid, benzenesulfonic acid, toluenesulfonic acid, camphorsulfonic acid, and similarly commonly known acceptable acids, provided that the rapamycin compound comprises an appropriate basic moiety. Provided that the rapamycin compound comprises an appropriate acid moiety, salts may be formed from organic bases and inorganic bases, for example, alkali metal salts (for example, sodium salt, lithium salt or potassium salt), alkaline earth metal salts, ammonium salts, alkylammonium salts containing one to six carbon atoms or dialkylammonium salts containing one to six carbon atoms in each alkyl group thereof, and trialkylammonium salts containing one to six carbon atoms in each alkyl group thereof.

The concentration of rapamycin compound added to the medium is not particularly limited, as long as production of regulatory T cells can be achieved by the method of the present invention, and the concentration is normally about 1 to 100 nM, preferably about 5 to 25 nM.

The timing of addition of rapamycin compound to the medium is not particularly limited, as long as production of regulatory T cells can be achieved by the method of the present invention; the rapamycin compound may be added from the start of cultivation, and the rapamycin compound may be added in the midst of cultivation. However, if the rapamycin compound is added from the start of cultivation, the proliferation of CD4⁺ T cells (particularly, CD25⁺CD4⁺ T cells) having a naive phenotype or T cells derived from the cells is suppressed, and the number of regulatory T cells obtained after cultivation sometimes decreases; therefore, it is preferable that the rapamycin compound be added in the midst of cultivation. In this case, the rapamycin compound is added to the medium, for example, 1 week after the start of cultivation or later, preferably 15 days after the start of cultivation or later. In particular, if CD25⁺CD4⁺ T cells having a naive phenotype are used, the rapamycin compound is preferably added to the medium 30 days after the start of cultivation or later. The rapamycin compound is preferably added to the medium, for example, until 100 days, preferably until 70 days, more preferably until 40 days, after the start of cultivation at the least.

The duration of cultivation in the presence of a rapamycin compound is not particularly limited, as long as production of regulatory T cells can be achieved by the method of the present invention, the duration is normally 1 to 100 days, for example, 3 to 70 days, preferably 5 to 50 days, more preferably 5 to 15 days. If the duration is too short, the effect of the rapamycin compound to induce the immunosuppressive function in CD4⁺ T cells having a naive phenotype or T cells derived from the cells, or to maintain the high immunosuppressive function of regulatory T cells, is sometimes not fully achieved; if the duration is too long, the proliferation of CD4⁺ T cells having a naive phenotype or T cells derived from the cells is suppressed, and the number of regulatory T cells obtained after cultivation sometimes decreases.

The cells obtained by cultivation in the presence of a rapamycin compound may be further cultured in the absence of the rapamycin compound. The conditions for the further cultivation can be the same as those described above, except that the rapamycin compound is not added; preferably, T cells are cultured in the absence of an antigen. By culturing T cells in the absence of an antigen, the proliferation thereof can be interrupted transiently. The duration of the further cultivation is not particularly limited, and the duration is normally 1 to 20 days, for example, 1 to 10 days, preferably 1 to 3 days. By this further cultivation, the rapamycin compound contaminated in the cells can more completely be removed.

By cultivation as mentioned above, CD4⁺ T cells of a naïve phenotype can proliferate, and can be expanded to 1000-fold or more in number over a long term of 30 days or more.

As a result of the cultivation, regulatory T cells can be obtained in the culture product.

More specifically, as shown in the below-mentioned Examples, the cells that can be obtained by the method of the present invention acquire the ability to inhibit activation of reactive T cell (immunosuppressive function) when they are cultured with reactive T cells and subjected to a T cell receptor-mediated stimulation. Furthermore, the obtained cell can also have the feature of unresponsiveness.

Importantly, when CD4⁺ T cells of a naive phenotype are cultured in the presence of an antigen according to the method of the present invention, the obtainable regulatory T cells can be specific to the antigen used for the cultivation.

Here, if CD25⁺CD4⁺ T cells having a naive phenotype are used in the method of the present invention, the obtainable regulatory T cells will have immunosuppressive function that depends on cell contact. Specifically, in this case, the obtainable cells have the capability of inhibiting the activation of reactive T cells when co-cultured with the reactive T cells under conditions that allow cell contact with the reactive T cells, and stimulated via a T cell receptor.

Meanwhile, if CD25⁻CD4⁺ T cells having a naive phenotype are used in the method of the present invention, the obtainable regulatory T cells will have immunosuppressive function that does not depend on cell contact. Specifically, in this case, the obtainable cells are capable of inhibiting the activation of reactive T cells when co-cultured with the reactive T cells, and stimulated via a T cell receptor, irrespective of cell contact with the reactive T cells.

Whether or not the obtained cells are functional as a regulatory T cell may be confirmed. For example, the obtained cells are cultured with a reactive T cells (e.g., total CD4⁺ T cell) and stimulated with an antigen, after which activation of the reactive T cells is determined. As the index of activation of reactive T cell, cell proliferation (e.g., [³H]-thymidine uptake) or production of cytokine (IL-2, IL-4, IFNγ etc.) is generally used. When activation of reactive T cells is inhibited as a result of the test, the obtained cells can be concluded to be functional as regulatory T cells.

At that time, it may be determined whether or not the immunosuppressive function possessed by the acquired cells is dependent on cell contact. For example, the acquired cells are cultured along with reactive T cells (e.g., total CD4⁺ T cells) and stimulated with an antigen, and the activation of the reactive T cells is measured, under two conditions: (A) conditions that allow cell contact, and (B) conditions that inhibit cell contact. Conditions (B) can be prepared by, for example, partitioning a chamber with a membrane having pores with sizes that allow the passage of liquid factors, but do not allow the passage of cells, into an upper section and a lower section (trans-well), placing reactive T cells in one section, and the acquired cells in the other section, and stimulating the cells in both sections with an antigen. If the test results show that the activation of the reactive T cells was suppressed only under conditions (A), it can be judged that the acquired cells have immunosuppressive function that depends on cell contact. If the activation of the reactive T cells was suppressed under both conditions (A) and (B), it can be judged that the acquired cells have immunosuppressive function that does not depend on cell contact.

Using, as an antigen to be used for stimulation, a combination of the same antigen as the antigen used in the production process of regulatory T cell and an antigen different from the antigen, whether or not the produced regulatory T cell is specific to the antigen can be confirmed.

Regulatory T cells that can be produced by the method of the present invention can have at least 1 kind (preferably 2 kinds) of phenotype selected from the group consisting of GITR positive (GITR⁺) and CTLA4 positive (CTLA4⁺). The phenotype is the same as that of naturally occurring regulatory T cells in periphery (e.g., CD45RA⁻CD25^{high+}CD4⁺ T cells and the like).

Regulatory T cells that can be produced by the method of the present invention can have a higher CD45RB expression level (CD45RB^{high+}) than naturally occurring regulatory T cells in periphery (e.g., CD45RA⁻CD25^{high+}CD4⁺ T cells in peripheral blood and the like). For example, in flowcytometry analysis using a fluorescence labeled anti-CD45RB antibody, the CD45RB expression level of regulatory T cells that can be produced by the method of the present invention can be normally about 1.5 to 20 times (preferably about 2 to 10 times (e.g., about 4 times)) that of CD45RA⁻CD25^{high+}CD4⁺ T cells in peripheral blood, under conditions that can produce a fluorescence intensity about 25 to 100 times (e.g., about 50 times) that obtained using an isotype control antibody, as the CD45RB expression by CD45RA⁻CD25^{high+}CD4⁺ T cells in peripheral blood. The expression level is compared by a flow cytometric analysis based on the comparison of the expression intensity at the peaks in a histogram in a graph wherein the vertical axis shows the number of cells, and the transverse axis shows the intensity of expression (fluorescence intensity).

Accordingly, regulatory T cells that can be produced by the method of the present invention can have at least 1 kind (preferably 2 kinds, more preferably 3 kinds) of phenotype selected from the group consisting of GITR⁺, CTLA4⁺ and CD45RB^{high+}.

Some of the phenotypes of regulatory T cells that can be produced by the method of the present invention are variable depending on the kind of CD4⁺ T cells having a naive phenotype for use in the method of the present invention (e.g., presence or absence of CD25 expression and the like), the timing of addition of rapamycin compound to the medium and the like.

For example, regulatory T cells that can be produced by the method of the present invention can be CD25-positive (CD25⁺), and the expression intensity thereof differs between cases where CD25⁺ T cells are used in the method of the present invention and cases where CD25⁻ T cells are used.

Accordingly, if CD25⁺CD4⁺ T cells having a naive phenotype are used in the method of the present invention, the obtainable regulatory T cells can have nearly the same level of CD25 expression as naturally occurring regulatory T cells in periphery (e.g., CD45RA⁻CD25^{high+}CD4⁺ T cells in peripheral blood and the like) (designated as CD25^{high+}). For example, in flowcytometry analysis using a fluorescence labeled anti-CD25 antibody, the CD25 expression level of regulatory T cells that can be produced with the use of CD25⁺CD4⁺ T cells having a naive phenotype in the method of the present invention can be normally about 1/6 to 6 times (preferably about 1/3 to 3 times (e.g., about 0.8 times)) that of CD45RA⁻CD25^{high+}CD4⁺ T cells in peripheral blood, under conditions that can produce a fluorescence intensity about 50 to 200 times (e.g., about 100 times) that obtained using an isotype control antibody, as the CD25 expression by CD45RA⁻CD25^{high+}CD4⁺ T cells in peripheral blood.

Meanwhile, if CD25⁻CD4⁺ T cells having a naive phenotype are used in the method of the present invention, the obtainable regulatory T cells can have a lower level of CD25 expression than naturally occurring regulatory T cells in periphery (e.g., CD45RA-CD25^{high+}CD4⁺ T cells in peripheral blood and the like) (CD25^{low+}). For example, under the same conditions as those described above, the CD25 expression level of regulatory T cells that can be produced with the use of CD25⁻CD4⁺ T cells having a naive phenotype in the method of the present invention can be normally about 1/15 to 1/2 times (preferably about 1/10 to 1/3 times (e.g., about 1/5 times)) that of CD45RA⁻CD25^{high+}CD4⁺ T cells in peripheral blood.

The CD62L expression intensity of regulatory T cells that can be produced by the method of the present invention also differ between cases where CD25⁺ T cells are used in the method of the present invention and cases where CD25⁻ T cells are used.

Accordingly, if CD25⁺CD4⁺ T cells having a naive phenotype are used in the method of the present invention, the obtainable regulatory T cells can have a lower level of CD62L expression than naturally occurring regulatory T cells in periphery (e.g., CD45RA⁻CD25^{high+}CD4⁺ T cells in peripheral blood and the like) (designated as CD62L^{low+}). For example, in flowcytometry analysis using a fluorescence labeled anti-CD62L antibody, the CD62L expression level of regulatory T cells that can be produced with the use of CD25⁺CD4⁺ T cells having a naive phenotype in the method of the present invention can be normally about 1/100 to 1/2 times (preferably about 1/50 to 1/3 times (e.g., about 1/6 times)) that of CD45RA-CD25^{high+}CD4⁺ T cells in peripheral blood, under conditions that can produce a fluorescence intensity about 100 to 500 times (e.g., about 300 times) that obtained using an isotype control antibody, as the CD62L expression by CD45RA⁻CD25^{high+}CD4⁺ T cells in peripheral blood.

Meanwhile, if CD25⁻CD4⁺ T cells having a naive phenotype is used in the method of the present invention, the obtainable regulatory T cells can have nearly the same level of CD62L expression as that of naturally occurring regulatory T cells in periphery (e.g., CD45RA-CD25^{high+}CD4⁺ T cells in peripheral blood and the like) (designated as CD62L^{high+}). For example, under the same conditions as those described above, the CD62L expression level of regulatory T cells that can be produced with the use of CD25⁻CD4⁺ T cells having a naive phenotype in the method of the present invention can be normally about 1/6 to 3 times (preferably about 1/4 to 2 times (e.g., about 1/3 times)) that of CD45RA⁻CD25^{high+}CD4⁺ T cells in peripheral blood.

Although the regulatory T cells that can be produced by the method of the present invention can be CD45RA-positive (CD45RA⁺), the expression intensity thereof differs between cases where CD25⁺ T cells are used in the method of the present invention and cases where CD25⁻ T cells are used.

Accordingly, if CD25⁺CD4⁺ T cells having a naive phenotype are used in the method of the present invention, the obtainable regulatory T cells can have a lower level of CD45RA expression than the CD25⁺CD4⁺ T cells having a naive phenotype used (e.g., CD45RA⁺CD25⁺CD4⁺ T cells in peripheral blood) (designated as CD45RA^{mid+}). For example, in flowcytometry analysis using a fluorescence labeled anti-CD45RA antibody, the CD45RA expression level of regulatory T cells that can be produced with the use of CD25⁺CD4⁺ T cells having a naive phenotype in the method of the present invention can be normally about 1/20 to 1/2 times (preferably about 1/10 to 1/3 times (e.g., about 1/6 times)) that of CD45RA⁺CD25⁺CD4⁺ T cells in peripheral blood, under conditions that can produce a fluorescence intensity about 100 to 1000 times (e.g., about 600 times) that obtained using an isotype control antibody, as the CD45RA expression by CD45RA⁺CD25⁺CD4⁺ T cells in peripheral blood.

Meanwhile, if CD25⁻CD4⁺ T cells having a naive phenotype are used in the method of the present invention, the obtainable regulatory T cells can have nearly the same level of CD45RA expression as that of CD25⁻CD4⁺ T cells having a naive phenotype used (e.g., CD45RA⁺CD25⁻CD4⁺ T cells in peripheral blood) (designated as CD45RA^{high+}). For example, in flowcytometry analysis using a fluorescence labeled anti-CD45RA antibody, the CD45RA expression level of regulatory T cells that can be produced with the use of CD25⁻CD4⁺ T cells having naive phenotype in the method of the present invention can be normally about 1/6 to 6 times (preferably about 1/3 to 3 times (e.g., about 1 times)) that of CD45RA⁺CD25⁻CD4⁺ T cells in peripheral blood, under conditions that can produce a fluorescence intensity about 100 to 1000 times (e.g., about 600 times) that obtained using an isotype control antibody, as the CD45RA expression by CD45RA⁺CD25⁻CD4⁺ T cells in peripheral blood. The CD45RA expression level of CD45RA⁺CD25⁺CD4⁺ T cells in peripheral blood is nearly the same as that of CD45RA⁺CD25⁻CD4⁺ T cells in peripheral blood (FIG. 1A).

The CD45RO expression intensity of regulatory T cells that can be produced by the method of the present invention can also differ between cases where CD25⁺ T cells are used in the method of the present invention and cases where CD25⁻ T cells are used.

Accordingly, if CD25⁻CD4⁺ T cells having a naive phenotype are used in the method of the present invention, the CD45RO expression by the obtainable regulatory T cells can be substantially negative (CD45RO⁻).

Meanwhile, if CD25⁺CD4⁺ T cells having a naive phenotype are used in the method of the present invention, CD45RO expression intensity can vary according to the timing of addition of rapamycin compound to the medium, duration of cultivation and the like.

Accordingly, in the method of the present invention, if CD25⁺CD4⁺ T cells having a naive phenotype are cultured in the presence of a rapamycin compound for a relatively long period (e.g., 50 days or more) from the start of cultivation, the CD45RO expression by the obtainable regulatory T cells can be substantially negative (CD45RO⁻).

Meanwhile, if a rapamycin compound is added to the medium from the midst of cultivation (e.g., 40 days after the start of cultivation), and the duration of cultivation in the presence of the rapamycin compound is relatively short (e.g., about 5 to 15 days), the CD45RO expression by the obtainable regulatory T cells can be at an intermediate level between CD25⁺CD4⁺ T cells having a naive phenotype (e.g., CD45RA⁺CD25⁺CD4⁺ T cells in peripheral blood) and naturally occurring regulatory T cells in periphery (e.g., CD45RA⁻CD25^{high+}CD4⁺ T cells in peripheral blood) (i.e., CD45RO^{mid+}). For example, in flowcytometry analysis using a fluorescence labeled anti-CD45RO antibody, the CD45RO expression level of regulatory T cells that can be obtained with the use of CD25⁺CD4⁺ T cells having a naive phenotype in the method of the present invention can be normally about 1/200 to 1/20 times (preferably about 1/100 to 1/30 times (e.g., about 1/50 times)) that of CD45RA⁻CD25^{high+}CD4⁺ T cells in peripheral blood, under conditions that can produce a fluorescence intensity about 200 to 800 times (e.g., about 400 times) that obtained using an isotype control antibody, as the CD45RO expression by CD45RA⁻CD25^{high+}CD4⁺ T cells in peripheral blood.

Accordingly, the regulatory T cells that can be produced by the method of the present invention can have at least 1 kind (preferably 2 kinds, more preferably 3 kinds, still more preferably 4 kinds) of phenotype selected from one of the groups (A) to (C) below:
(A) CD45RO^{mid+}, CD45RA^{mid+}, CD62L^{low+}, and CD25^{high+}
(B) CD45RO⁻, CD45RA^{mid+}, CD62L^{low+}, and CD25^{high+}
(C) CD45RO⁻, CD45RA^{high+}, CD62L^{high+}, and CD25^{low+}.

Here, the regulatory T cells that can be produced with the use of CD25⁺CD4⁺ T cells having a naive phenotype in the method of the present invention can have at least 1 kind (preferably 2 kinds, more preferably 3 kinds, still more preferably 4 kinds) of phenotype selected from one of the groups (A) and (B) above. Meanwhile, the regulatory T cells that can be produced with the use of CD25⁻CD4⁺ T cells having a naive phenotype in the method of the present invention can have at least 1 kind (preferably 2 kinds, more preferably 3 kinds, still more preferably 4 kinds) of phenotype selected from the group (C) above.

The present invention also provides regulatory T cells having at least 1 kind (preferably 2 kinds, more preferably 3 kinds, still more preferably 4 kinds) of phenotype selected from one of the groups (A) to (C) below:
(A) CD45RO^{mid+}, CD45RA^{mid+}, CD62L^{low+}, and CD25^{high+}
(B) CD45RO⁻, CD45RA^{mid+}, CD62L^{low+}, and CD25^{high+}
(C) CD45RO⁻, CD45RA^{high+}, CD62L^{high+}, and CD25^{low+}.

The regulatory T cells can further have at least 1 kind (preferably 2 kinds, more preferably 3 kinds) of phenotype selected from the group consisting of GITR⁺, CTLA4⁺ and CD45RB^{high+}. These phenotypes are as defined above. The regulatory T cells are preferably isolated and purified. The regulatory T cells can be produced by the above-described method of the present invention.

### (2. Immunoregulator comprising regulatory T cells)

The regulatory T cells which can be produced by the method of the present invention has a strong immunosuppressive function. Therefore, when an immunoreaction is abnormally accelerated in vivo for some reason, when an undesirable immunoreaction is ongoing in vivo, or when an undesirable immune response is predicted to occur in the future, or the like, the regulatory T cells are administered to a patient, whereby, in the body of patient, an abnormally-accelerated immunoreaction can be suppressed, an undesirable immunoreaction can be suppressed, or an undesirable immunoreaction can be avoided.

For example, prior to organ transplantation from an allogenic donor, recipient-derived CD4⁺ T cells of a naive phenotype are cultured in the presence of rapamycin compound and donor-derived cells to give a regulatory T cell specific to the donor-derived cells. Then, the regulatory T cells are administered to a recipient before and after the transplantation to induce immune tolerance to a graft, whereby rejection reaction to the graft can be avoided and engraftment of the graft can be promoted. Accordingly, the immunomodulator of the present invention can avoid side effects caused by the existing immunosuppressants, bacterial or viral infection, particularly, recurrent hepatitis C after liver transplantation in hepatitis C patients.

In addition, in patients with an autoimmune disease, patient-derived CD4⁺ T cells of a naive phenotype are cultured in the presence of rapamycin compound and a causative antigen of the autoimmune disease, whereby regulatory T cells specific to the causative antigen are produced. By administration of the regulatory T cells to the patient, the autoimmune reaction can be suppressed.

Moreover, in patients with an allergic disease, patient-derived CD4⁺ T cells of a naive phenotype are cultured in the presence of rapamycin compound and a causative antigen of the allergy, whereby regulatory T cells specific to the causative antigen are produced. By administration of the regulatory T cell to the patient, the allergic reaction can be suppressed.

In an infertility patient, patient-derived CD4⁺ T cells of a naive phenotype is cultured in the presence of rapamycin compound and partner-derived cells, whereby regulatory T cells specific to the partner-derived cells (or antigen) is produced. By administration of the regulatory T cell to the patient, the immune tolerance to the partner-derived cells (or antigen) or a fetus having the partner-derived antigen is induced, the rejection reaction to the fetus is avoided, and the maintenance of pregnancy can be facilitated.

Accordingly, the present invention provides an immunomodulator containing the regulatory T cells which can be produced by the above-mentioned method as an active ingredient. The agent can be used for immunosuppression. The immunomodulator of the present invention can be used for the prophylaxis or treatment of the rejection of organ transplantation, allergic diseases (pollinosis, food allergy, drug allergy, asthma, atopic dermatitis, eczema, food hypersensitivity, urticaria, allergic rhinitis, allergic conjunctivitis), autoimmune diseases (polymyositis, chronic rheumatism, systemic lupus erythematosus, systemic sclerosis, bullous diseases, cutaneous lupus erythematosus, psoriasis, Crohn's disease, ulcerative colitis, autoimmune hepatitis, multiple sclerosis, type 1 diabetes etc.), graft-versus-host disease (GVHD), infertility and the like.

The immunomodulator of the present invention can be produced as an oral/parenteral preparation by mixing the effective amount of the above-mentioned regulatory T cells with a pharmaceutically acceptable carrier, and the like, according to a conventional method. The immunomodulator of the present invention is generally produced as a parenteral preparation such as injection, suspension, drip infusion and the like. As the carrier to be contained in the parenteral preparation, for example, an aqueous solution for injection such as physiological saline, isotonic solution containing glucose or other auxiliary agents (e.g., D-sorbitol, D-mannitol, sodium chloride and the like) and the like can be mentioned. The immunomodulator of the present invention may be blended with, for example, buffers (e.g., phosphate buffer, sodium acetate buffer), analgesics (e.g., benzalkonium chloride, procaine hydrochloride and the like), stabilizers (e.g., human serum albumin, polyethylene glycol and the like), preservatives, antioxidants and the like.

The preparation obtained in this manner is safe and low toxic. Therefore, it can be administered, for example, to the aforementioned mammals such as human and the like.

While the dose of the regulatory T cells of the present invention varies depending on the subject of administration, target organ, symptom, administration method and the like, for example, in the case of parenteral administration to an adult patient (body weight 60 Kg), an effective amount with the upper limit being about 6 x 10⁹ a day is generally administered conveniently. For other animals, an amount converted based on the amount per 60 kg can be administered.

### (3. Kit for production of regulatory T cells)

The present invention also provides a kit for production of regulatory T cells, comprising an antibody for preparing CD4⁺ T cells having a naive phenotype and a rapamycin compound. The antibody may be labeled with a fluorescent dye, magnetic beads and the like. The antibody is exemplified by specific antibodies against antigens expressed specifically in CD4⁺ T cells having a naive phenotype (e.g., anti-CD4 antibody, anti-CD45RA antibody, anti-CD45RB antibody, anti-CD62L antibody, anti-CD38 antibody and the like). In order to further isolate and purify CD25⁺ cells or CD25⁻ cells in CD4⁺T cells having a naive phenotype, the antibody can further include anti-CD25 antibody. The kit can further comprise various reagents that can be used in the above-described method (antigens, T cell proliferation factor, media, medium additives, serum, reaction vessels, instructions bearing the protocol for the method of the present invention for producing regulatory T cells and the like). By using the kit, regulatory T cells can conveniently be produced according to the method described above.

### (4. Agent for inducing regulatory T cells)

As described above, by using a rapamycin compound in the method of producing regulatory T cells according to the present invention, it is possible to induce immunosuppressive function in CD4⁺ T cells having a naive phenotype or T cells derived from the cells to acquire regulatory T cells. Accordingly, the present invention provides an agent for inducing regulatory T cells from CD4⁺ T cells having a naive phenotype, comprising a rapamycin compound. The CD4⁺ T cells can be CD25⁺ or CD25⁻, and are preferably CD25⁻. The agent of the present invention can contain as an active ingredient a rapamycin compound alone, or in a form of a mixture with an optionally chosen other active ingredient. The agent of the present invention can be produced by an optionally chosen method commonly known in the technical field of pharmaceutical preparation with an active ingredient mixed with one or more pharmaceutically acceptable carriers. Examples of the carrier include physiological isotonic solutions (physiological saline, above-described basal media, isotonic solutions comprising glucose or other auxiliaries (e.g., D-sorbitol, D-mannitol, sodium chloride and the like) and the like), buffer agents (e.g., phosphate buffer solution, sodium acetate buffer solution), stabilizers (e.g., human serum albumin, polyethylene glycol and the like), preservatives, antioxidants, excipients, antiseptics, binders, solubilizers, non-ionic surfactants and the like. The agent of the present invention is used in the form of isotonic aqueous solutions, powder and the like to be added to the medium for use in the production method of present invention and the like.

A rapamycin compound can also be prepared as a pharmaceutical preparation by a method known per se, and can be used to induce regulatory T cells from CD4⁺ T cells having a naive phenotype in vivo. A pharmaceutical preparation comprising a rapamycin compound can contain as an active ingredient a rapamycin compound alone, or in a form of as a mixture with an optionally chosen other active ingredient for treatment. These pharmaceutical preparations are produced by mixing an active ingredient with one or more pharmaceutically acceptable carriers according to an optionally chosen method well known in the technical field of pharmaceutical preparation.

The route for administration is desirably the therapeutically most effective one, and is exemplified by oral administration and parenteral administration, for example, intravenous administration. Dosage forms include tablets, powders, granules, syrups, injections and the like. Liquid preparations suitable for oral administration, for example, syrups, can be produced using water, sugars such as sucrose, sorbit, and fructose, glycols such as polyethylene glycol and propylene glycol, oils such as sesame oil, olive oil, and soybean oil, antiseptics such as p-hydroxybenzoic acid esters, flavors such as strawberry flavor and peppermint, and the like. Tablets, powders, granules and the like can be produced using excipients such as lactose, glucose, sucrose, and mannit, disintegrants such as starch and sodium alginate, lubricants such as magnesium stearate and talc, binders such as polyvinyl alcohol, hydroxypropylcellulose, and gelatin, surfactants such as fatty acid esters, plasticizers such as glycerin, and the like.

A preparation suitable for parenteral administration preferably consists of a sterile aqueous agent comprising an active compound that is isotonic to the recipient's blood. For example, in the case of an injection, a solution for injection is prepared using a carrier consisting of a salt solution, a glucose solution, or a mixture of saline and glucose solution and the like. These parenteral preparations may be supplemented with one or more auxiliary ingredients selected from among the diluents, antiseptics, excipients, disintegrants, lubricants, binders, surfactants, plasticizers and the like mentioned for exemplification for use in oral preparations.

The dosage and frequency of administration of the rapamycin compound vary depending on dosage form, patient's age and body weight, nature or seriousness of the symptoms to be treated; normally, in the case of parenteral administration such as intravenous administration, about 0.6 to 5 mg per adult (assuming a body weight of 60 kg) is administered once to several times a day. In the case of oral administration, about 2 to 15 mg per adult (assuming a body weight of 60 kg) is administered once to several times a day. In the case of other animals, an amount calculated per 60 kg can be administered. However, these dosages and frequencies of administration vary depending on the aforementioned various conditions.

### (5. Agent for maintaining the immunosuppressive function of regulatory T cells)

As described above, by using a rapamycin compound in the method of producing regulatory T cells according to the present invention, it is possible to suppress the reduction in the immunosuppressive function of regulatory T cells that can accompany cell proliferation at high ratios, to maintain the immunosuppressive function of regulatory T cells at high levels, or to enhance the immunosuppressive function of regulatory T cells. Accordingly, the present invention provides an agent for maintaining the immunosuppressive function of regulatory T cells in producing regulatory T cells by culturing CD4⁺ T cells having a naive phenotype to obtain regulatory T cells, comprising a rapamycin compound. The CD4⁺ T cells can be CD25⁺ or CD25⁻, and are preferably CD25⁺. Here, "maintenance of the immunosuppressive function of regulatory T cells" includes suppression of the reduction in the immunosuppressive function of regulatory T cells, maintenance of the immunosuppressive function of regulatory T cells at high levels, and enhancing the immunosuppressive function of regulatory T cells. The agent of the present invention can comprise a pharmaceutically acceptable carrier, in addition to an effective amount of rapamycin compound. The carrier may be as described in the foregoing section (4. Agent for inducing regulatory T cells). The agent of the present invention is used in the form of isotonic aqueous solutions, powders and the like to be added to the medium for use in the method of production according to the present invention and the like.

### (6. Screening method for compound capable of inducing regulatory T cells)

The present invention provides a screening method for a compound capable of inducing regulatory T cells, comprising the following steps:
(1) a step for culturing CD4⁺ T cells having a naive phenotype in the presence of a test compound, and isolating T cells from the culture product;
(2) a step for evaluating the immunosuppressive function of the T cells obtained in the step (1);
(3) a step for obtaining the test compound as a compound capable of inducing regulatory T cells if the results of the evaluation in the step (2) show that the T cells isolated in the step (1) have immunosuppressive function.

In the screening method of the present invention, first, CD4⁺ T cells having a naive phenotype are cultured in the presence of a test compound, and T cells are isolated from the culture product (step 1). The CD4⁺ T cells can be CD25⁺ or CD25⁻, and are preferably CD25⁻. The test compound may be any commonly known compound or a novel compound; such compounds include, for example, nucleic acids, glucides, lipids, proteins, peptides, organic low-molecular compounds, compound libraries prepared using combinatorial chemistry technology, random peptide libraries prepared by solid phase synthesis or the phage display method, natural components derived from microorganisms, animals, plants, marine organisms, and the like.

As described above, by using a rapamycin compound in the method of producing regulatory T cells according to the present invention, immunosuppressive function is induced in CD4⁺ T cells having a naive phenotype or T cells derived from the cells. Therefore, immunosuppressive compounds like rapamycin compounds can be suitable test compounds. An immunosuppressive compound refers to a compound capable of suppressing immune responses in vitro or in vivo. Examples of the immunosuppressive compound, other than rapamycin compounds, include FK506, cyclosporine A and the like. Because regulatory T cells can be induced at high efficiency using a rapamycin compound, macrolide compounds (rapamycin compound, FK506, cyclosporine A and the like), out of immunosuppressive compounds, can be suitable test compounds. A macrolide compound refers to a compound having a 14- to 16-membered lactone ring. Derivatives obtained by chemically modifying immunosuppressive compounds to substitute groups at substitutable positions with other groups can also be suitable test compounds.

The culture conditions in the step 1 may be as described in the foregoing section (1. Method of producing regulatory T cells), except that the cultivation may be performed in the absence of a rapamycin compound.

Next, the immunosuppressive function of the T cells obtained in the step 1 is evaluated (step 2). The immunosuppressive function of T cells is evaluated by, for example, culturing the T cells along with reactive T cells (e.g., total CD4⁺ T cells) under conditions that allow cell contact, stimulating the cells with an antigen, and measuring the degree of suppression of the activation of the reactive T cells. As indicators of the activation of the reactive T cells, cell proliferation (e.g., [³H] thymidine uptake) and cytokine (IL-2, IL-4, IFNγ and the like) production are normally used. As the antigen for stimulation, the same antigen as the antigen used in producing T cells in the step 1 and different antigens can be used.

Next, if the results of the evaluation in the step 2 show that the T cells obtained in the step 1 have immunosuppressive function, the above-described test compound is acquired as a compound capable of inducing regulatory T cells (step 3). A compound obtained by the screening method of the present invention is capable of inducing regulatory T cells, in place of a rapamycin compound, in the above-described method of producing regulatory T cells according to the present invention, and is useful in pharmaceutical and medical fields using regulatory T cells, and is also useful as an immunosuppressive drug based on the new mechanism of inducing regulatory T cells from CD4⁺ T cells having a naive phenotype, or as a seed for the development thereof.

### (7. Screening method for a compound capable of increasing the efficiency of production of regulatory T cells)

The present invention provides a screening method for a compound capable of increasing the efficiency of production of regulatory T cells, comprising the following steps:
(1) a step for culturing CD25⁺CD4⁺ T cells having a naive phenotype in the presence of a test compound to obtain regulatory T cells;
(2) a step for evaluating the number and/or immunosuppressive function of the regulatory T cells obtained in the step (1);
(3) a step for comparing the number and/or immunosuppressive function of regulatory T cells evaluated in the step (2) with the number and/or immunosuppressive function of regulatory T cells obtained by culturing CD25⁺CD4⁺ T cells having a naive phenotype in the absence of the test compound;
(4) a step for obtaining a test compound that has increased the number and/or immunosuppressive function of regulatory T cells as a compound capable of increasing the efficiency of production of regulatory T cells.

"Increasing the efficiency of production of regulatory T cells" means increasing the number and/or immunosuppressive function of regulatory T cells that can be obtained in the production of regulatory T cells.

In the screening method of the present invention, first, CD25⁺CD4⁺ T cells having a naive phenotype are cultured in the presence of a test compound, and regulatory T cells are acquired from the culture product (step 1). The test compound can be as described in the foregoing section (6. screening method for a compound capable of inducing regulatory T cells).

As described above, by using a rapamycin compound in the method of producing regulatory T cells of the present invention, it is possible to suppress the reduction in the immunosuppressive function of regulatory T cells that can accompany cell proliferation at high ratios, to maintain the immunosuppressive function of regulatory T cells at high levels, or to enhance the immunosuppressive function of regulatory T cells. Therefore, immunosuppressive compounds like rapamycin compounds can be suitable test compounds. Examples of the immunosuppressive compound, other than rapamycin compounds, include FK506, cyclosporine A and the like. Because regulatory T cells can be induced at high efficiency by using a rapamycin compound, macrolide compounds (rapamycin compound, FK506, cyclosporine A and the like) out of immunosuppressive compounds can be suitable test compounds. A macrolide compound refers to a compound having a 14- to 16-membered lactone ring. Derivatives obtained by chemically modifying immunosuppressive compounds to substitute groups at substitutable positions with other groups can also be suitable test compounds.

The cell culture conditions in the step 1 may be as described in the foregoing section (1. Method of producing regulatory T cells), except that the cultivation can be performed in the absence of a rapamycin compound.

Next, the number and/or immunosuppressive function of the regulatory T cells obtained in the step 1 is evaluated (step 2). The number of regulatory T cells can be determined by a method known per se. The immunosuppressive capability of regulatory T cells is evaluated by, for example, culturing the regulatory T cells along with reactive T cells (e.g., total CD4⁺ T cells) under conditions that allow cell contact, stimulating the cells with an antigen, and measuring the degree of suppression of the activation of the reactive T cells. As indicators of the activation of the reactive T cells, cell proliferation (e.g., [³H] thymidine uptake) and cytokine (IL-2, IL-4, IFNγ and the like) production are normally used. As the antigen for stimulation, the same antigen as the antigen used in producing regulatory T cells and different antigens can be used.

Next, the number and/or immunosuppressive function of regulatory T cells evaluated in the step 2 is compared with the number and/or immunosuppressive function of regulatory T cells obtained by culturing CD25⁺CD4⁺ T cells having a naive phenotype in the absence of the test compound (step 3). The comparison of the number and/or immunosuppressive function is preferably made on the basis of the presence or absence of a significant difference. Although the number and/or immunosuppressive function of the regulatory T cells obtained in the absence of the test compound may be a value measured before or simultaneously with the measurement of the number and/or immunosuppressive function of the regulatory T cells obtained in the presence of the test compound, it is preferable, from the viewpoint of experimental accuracy and reproducibility, that the number and/or function be a simultaneously measured value.

As a result of the comparison, a test compound that has increased the number and/or immunosuppressive function of the regulatory T cells is acquired as a compound capable of increasing the efficiency of production of the regulatory T cells (step 4). A compound obtained by the screening method of the present invention is capable of increasing the efficiency of production of regulatory T cells in the above-described method of producing regulatory T cells according to the present invention, in place of a rapamycin compound, and is therefore useful in pharmaceutical and medical fields using regulatory T cells, and is also useful as a seed for the development of immunosuppressants based on a new mechanism.

### (8. Method of inducing CD25⁺CD4⁺ T cells having a naïve phenotype)

The present invention provides a method of inducing CD25⁺CD4⁺ T cells having a naive phenotype in vivo, comprising the following steps:
(1) a step for performing liver transplantation on a mammal;
(2) a step for confirming the induction of CD25⁺CD4⁺ T cells having a naive phenotype in peripheral tissue of the mammal.

Mammals for use in the method of induction according to the present invention include the above-described mammals. The age of the mammal is not particularly limited, and the mammal can be a juvenile or an adult; however, because a larger number of CD25⁺CD4⁺ T cells can be induced at younger ages, the mammal is preferably a juvenile. A juvenile and an adult refer to a sexually immature individual and a sexually mature individual, respectively. In humans, it is common practice that individuals under 18 years are handled as juveniles, and individuals 18 years or older are handled as adults.

In the induction method of the present invention, liver transplantation is performed on a mammal (step 1). The genotype of the liver for transplantation is not particularly limited, and can be immunologically self (i.e., syngeneic) or immunologically non-self (i.e., allogenic or heterogenic), and is preferably immunological non-self, more preferably allogenic.

Liver transplantation includes orthotropic liver transplantation and heterotropic liver transplantation, and orthotropic liver transplantation is preferable. Orthotropic liver transplantation is a method wherein total extirpation of the recipient's liver is followed by reconstruction of vasculature of the graft liver at the normal anatomical position. Heterotropic liver transplantation is a method wherein a graft liver is intraperitoneally transplanted to a position different from the normal position of the liver while preserving the host's liver. The method of liver transplantation is known per se; see, for example, Tanaka K, Ann Surg., 217, 82-91, 1993 and the like.

When liver transplantation is performed, CD25⁺CD4⁺ T cells having a naive phenotype are induced in the host's peripheral tissue, and the number of CD25⁺CD4⁺ T cells having a naive phenotype in the peripheral tissue increases. Usually, 1 to 3 months after liver transplantation, the number of CD25⁺CD4⁺ T cells having a naive phenotype in peripheral tissue (e.g., peripheral blood and the like) increases up to about 1.2 to 5 times.

Next, the induction of CD25⁺CD4⁺ T cells having a naive phenotype in peripheral tissue of the mammal receiving the liver transplantation is confirmed (step 2). Usually, the number (or ratio) of CD25⁺CD4⁺ T cells having a naive phenotype in peripheral tissue (e.g., peripheral blood and the like) is measured, the number (or ratio) is compared with the cell number (or ratio) before the liver transplantation, and an increase in the number is confirmed. Detection of the number of CD25⁺CD4⁺ T cells having a naive phenotype (or ratio) can be performed on the basis of, for example, the phenotype of CD25⁺CD4⁺ T cells having a naive phenotype, using a specific antibody capable of detecting the phenotype and the like, by flowcytometry and the like.

Because the above-described method makes it possible to induce a large number of CD25⁺CD4⁺ T cells having a naive phenotype, which are starting materials for the above-described method of producing regulatory T cells according to the present invention, a large number of regulatory T cells can be induced from a small volume of sample (peripheral blood and the like) in the above-described production of regulatory T cells.

### (9. Screening method for a compound capable of increasing the number of CD25⁺CD4⁺ T cells having a naive phenotype)

The present invention provides a screening method for a compound capable of increasing the number of CD25⁺CD4⁺ T cells having a naive phenotype in vivo, comprising the following steps:
(1) a step for performing liver transplantation on a non-human mammal;
(2) a step for administering a test compound to the mammal of the step (1);
(3) a step for evaluating the number of CD25⁺CD4⁺ T cells having a naive phenotype in peripheral tissue of the mammal in the step (2);
(4) a step for comparing the number of CD25⁺CD4⁺ T cells having a naive phenotype evaluated in the step (3) with the number of CD25⁺CD4⁺ T cells having a naive phenotype in peripheral tissue of a non-human mammal that has undergone liver transplantation, but does not receive the test compound;
(5) a step for selecting a compound that has increased the number of CD25⁺CD4⁺ T cells having a naive phenotype in peripheral tissue.

The mammal used in the screening method of the present invention may be as described in the foregoing section (8. Method of inducing CD25⁺CD4⁺ T cells having a naive phenotype).

In the screening method of the present invention, first, liver transplantation is performed on a mammal (step 1). The embodiment of liver transplantation is as described in the foregoing section (8. Method of inducing CD25⁺CD4⁺ T cells having a naive phenotype).

Next, a test compound is administered to the mammal that has undergone the liver transplantation (step 2). The test compound is as described in the foregoing section (6. screening method for a compound capable of inducing regulatory T cells).

Next, the number of CD25⁺CD4⁺ T cells having a naive phenotype in periphery of the mammal of the step 2 is evaluated (step 3). Usually, the number (or ratio) of CD25⁺CD4⁺ T cells having a naive phenotype in peripheral tissue (e.g., peripheral blood and the like) is measured, the cell number (or ratio) is compared with the cell number (or ratio) before the liver transplantation, and an increase in the number is confirmed. Detection of the number (or ratio) of CD25⁺CD4⁺ T cells having a naive phenotype is performed on the basis of, for example, the phenotype of CD25⁺CD4⁺ T cells having a naive phenotype, using a specific antibody capable of detecting the phenotype and the like, by flowcytometry and the like. Timing of the evaluation is preferably 1 to 3 months after the liver transplantation.

Next, the number of CD25⁺CD4⁺ T cells having a naive phenotype evaluated in the step 3 is compared with the number of CD25⁺CD4⁺ T cells having a naive phenotype in periphery of a mammal that has undergone liver transplantation, but does not receive the test compound (step 4). The comparison of cell number is preferably made on the basis of the presence or absence of a significant difference. Although the number of CD25⁺CD4⁺ T cells having a naive phenotype in periphery of a mammal that has not received the test compound may be a value measured before or simultaneously with the measurement of the number of CD25⁺CD4⁺ T cells having a naive phenotype in periphery of a mammal that has received the test compound, it is preferable, from the viewpoint of experimental accuracy and reproducibility, that the number be a simultaneously measured value.

As a result of the comparison, a compound that has increased the number of CD25⁺CD4⁺ T cells having a naive phenotype in periphery is selected (step 5). The compound is capable of enhancing the induction of CD25⁺CD4⁺ T cells having a naive phenotype in peripheral tissue due to liver transplantation. Because the compound makes it possible to induce a large number of CD25⁺CD4⁺ T cells having a naive phenotype, which are starting materials for the above-described method of producing regulatory T cells according to the present invention, a large number of regulatory T cells can be induced from a small volume of sample (peripheral blood and the like) in the above-described production of regulatory T cells.

The present invention is explained in more detail in the following by referring to Examples, which are not to be construed as limitative.

### Examples

### [Example 1]

### [1] Material and method

### (cell preparation)

Venous blood was obtained from healthy volunteers (human). Peripheral blood mononuclear cell (PBMC) was isolated by Ficoll-Hypaque (Amersham Biosciences-Uppsala) density gradient centrifugation. The cells were stained with allophycocyanin (APC)-conjugated anti-CD4 monoclonal antibody (mAb), phycoerythrin (PE)-conjugated anti-CD25 mAb, and fluorescein isothiocyanate (FITC)-conjugated anti-CD45RA mAb for 30 min at 4°C in the dark, after which the following six different cell fractions were isolated using a BD FACSAria cell sorter (Becton Dickinson)(see Fig. 1A).
(a) CD45RA⁺CD25⁺CD4⁺ T cell
(b) CD45RA⁻CD25^{high+}CD4⁺ T cell
(c) CD45RA⁻CD25^{low+}CD4⁺ T cell
(d) CD45RA⁻CD25⁻CD4⁺ T cell
(e) CD45RA⁺CD25⁻CD4⁺ T cell, and
(f) total-CD4⁺ T cell

The cell fractions shown in the aforementioned (a)-(e) were isolated according to the definition shown in Fig. 1A.

All of the mAbs were purchased from Becton Dickinson. The purity of each fraction was >98%. For cell separation to perform a functional assay, CD4⁺ cells after removing the cells bound to a plate were positively isolated using anti-CD4 mAb microbeads and magnetic cell sorting (MACS) system magnetic columns (Miltenyi Biotec GmbH). The purity of the CD4⁺ cell was >95%.

### (FACS analysis)

PBMC derived from the subjects, or expanded T cell line were incubated with various antibodies for 30 min at 4°C in the dark, washed, and analyzed using a BD FACSAria cell sorter and a FACSDiVA software (Becton Dickinson). The antibodies used were perCP-conjugated anti-CD4 mAb, biotin-conjugated anti-CD25 mAb, FITC-conjugated anti-CD45RA mAb, PE-conjugated anti-CD45RB mAb, anti-CD45RO mAb, anti-CD62L mAb, anti-CD38 mAb, anti-GITR mAb, and anti-TNFRII mAb. For intracellular staining with CTLA-4, the cells were subjected to cell surface staining, fixation, and permeabilization with Cytofix/Cytoperm solution (Becton Dickinson), and then intracellularly stained with PE-conjugated anti-CTLA-4.

All mAbs and streptavidin-APC were purchased from Beckton Dickinson, Immunotech, or Genzyme/Techne.

### (cell cultivation)

CD45RA⁺CD25⁺CD4⁺ T cells or CD45RA⁺CD25⁻CD4⁺ T cells (5 x 10^4 cells/well) separated as described above were stimulated with anti-human CD3 and CD28 conjugate beads in a ratio of 4 beads per cell in a 96-well round-bottomed culture plate containing 200 µl per well of a proliferation medium (AlyS505N (produced by Cell Science & Technology Institute) supplemented with 2% human AB serum) in the presence of human IL-2 (1000 U/ml, produced by Cell Science & Technology Institute). Rapamycin (20 nM, produced by Sigma) was added as appropriate. Every 3 to 4 days, half of the medium was replaced with a fresh proliferation medium containing IL-2. If necessary, the cells were separated and repeatedly re-stimulated with anti-CD3/CD28 antibody conjugated beads every 10 to 14 days. Two days before functional assay, the anti-CD3/CD28 antibody conjugated beads were removed magnetically, and the cells were cultured along with 100 U/ml IL-2 in the absence of rapamycin. After 3 times of washing, the cells were used for functional assay.

### (Suppression assay)

The indicated number of CD45RA⁺CD25⁺CD4⁺ T cells just isolated from human peripheral blood, expanded CD45RA⁺CD25⁺CD4⁺ T cell lines, or expanded CD45RA⁺CD25⁻CD4⁺ T cell line, alone or along with self responder CD4⁺ T cells (5 x 10^4 cells/well), were stimulated with radiated (40 Gy) allogenic PBMC (1 x 10^5 cells/well) in a round-bottomed 96-well microplate containing a proliferation medium. The culture product was incubated for 7 days, and labeled with [³H] thymidine (2 µCi/well) during the last 16 hours. Subsequently, the cells were recovered, and [³H] thymidine uptake was measured using 1450 Microbeta TriLux (Wallac).

### (quantitative RT-PCR)

Total RNA was extracted from the CD4⁺ T cell fraction using Isogen reagent (Nippon Gene) according to the instruction provided by the manufacturer, and incubated for 1 hr at 37°C in 20 µl of reaction solution containing 10 mM DTT, 0.5 mM dNTPs, 1 x M-MLV RT buffer, 40 ng of random primer p(dN)6, 6U of ribonuclease inhibitor, and 40U of M-MLV reverse transcriptase (Invitrogen), followed by heating for 10 min at 70°C, whereby cDNA was synthesized. The expression level of FOXP3 mRNA was quantified by real-time PCR using ABI/PRISM7700 sequence detection system (Applied Biosystems) and QuantiTect Probe PCR kit (Quiagen). FOXP3-specific primers and intrinsic fluorescent Taqman probe were designed as described (Int Immunol., 16, 1643-1656, 2004). 20x primers and a probe mixture of HPRT were purchased from Applied Biosystems. The expression level of FOXP3 mRNA was normalized against that of mRNA of a house-keeping gene HPRT. The data is expressed with average values from triplicate-wells. The standard deviation was <5%.

### [2] Results

### (Expression of FOXP3 mRNA)

The expression levels of FOXP3 for the following six different cell fractions were measured.
(a) CD45RA⁺CD25⁺CD4⁺ T cell
(b) CD45RA⁻CD25^{high+}CD4⁺ T cell
(c) CD45RA⁻CD25^{low+}CD4⁺ T cell
(d) CD45RA⁻CD25⁻CD4⁺ T cell
(e) CD45RA⁺CD25⁻CD4⁺ T cell, and
(f) total-CD4⁺ T cell

These cells were isolated from PBMC according to the gate shown in Fig. 1A using a cell sorter. CD45RA⁻CD25^{high+}CD4⁺(b) and CD45RA⁺CD25⁺CD4⁺(a) T cell fraction showed higher expression levels of FOXP3 as compared to CD45RA⁻CD25^{low+}CD4⁺(c), CD45RA⁻CD25⁻CD4⁺(d) , CD45RA⁺CD25⁻CD4⁺(e), and total CD4⁺(f) T cell fraction (Fig. 1B) . Although CD45RA⁺CD25⁺CD4⁺ cell fraction had been considered to differ from Tregs, it showed a higher expression level of FOXP3 than even that of CD45RA⁻CD25^{high+}CD4⁺ cell fraction. In the CD45RA⁺CD25⁻CD4⁺ T cell fraction, FOXP3 gene was hardly expressed, and therefore, it was considered that a regulatory T cell was not present. Two or more tests were run for different individuals, and similar results were obtained.

### (Phenotype of CD45RA⁺CD25⁺CD4⁺ T cell and CD45RA⁺CD25⁻CD4⁺ T cell)

CD45RA⁺CD25⁺CD4⁺(a), CD45RA⁻CD25^{high+}CD4⁺(b), and CD45RA⁺CD25⁻CD4⁺(e) T cell (see Fig. 1A) were investigated as to naive/memory cell phenotype, and intracellular CTLR-4 and cell surface TNFRII (Fig.2). The CD45RA⁺CD25⁻CD4⁺ T cells were found to be CD45RO^{low+}, CD45RB^{high+}, CD62L^{high+} and CD38⁺; a typical naive phenotype was confirmed. Meanwhile, CD45RA-CD25^{high+}CD4⁺(b) T cells, which are regulatory T cells in periphery, were found to be CD45RO⁺, CD45RB^{low+}, CD62L^{low+} and CD38⁻. The expression intensities of various markers in CD45RA⁻CD25^{high+}CD4⁺ T cells in peripheral blood, compared with the fluorescence intensity obtained with the control antibody, were as follows, respectively; CD45RO: about 400 times, CD45RB: about 50 times, CD62L: about 300 times. This result shows that CD45RA⁻CD25^{high+}CD4⁺ T cells have a memory type phenotype. CD45RA⁺CD25⁺CD4⁺ T cells were CD45RO^{low+}, CD45RB^{high+}, CD62L^{high+} and CD38⁺; this phenotype was equal to that of CD45RA⁺CD25⁻CD4⁺ T cells. CD45RA⁺CD25⁻CD4⁺ T cells exhibited almost no intracellular CTLA-4 and TNFRII. Unlike this cell fraction, CD45RA⁻CD25^{high+}CD4⁺ T cells and CD45RA⁺CD25⁺CD4⁺ T cells exhibited intracellular CTLA-4 and TNFRII specific for regulatory T cells. The CD25 expression intensity of CD45RA⁻CD25^{high+}CD4⁺ T cells was about 100 times the fluorescence intensity obtained with the control antibody, and the CD45RA expression intensity of CD45RA⁺CD25⁺CD4⁺ T cells was about 600 times the fluorescence intensity obtained with the use of control antibody. Two or more experiments were performed for different individuals, and same results were obtained.

### (Functional property of expanded CD45RA⁺CD25⁺CD4⁺ T cell)

CD45RA⁺CD25⁺CD4⁺ T cells freshly isolated from periphery slightly suppressed the proliferation of CD4⁺ T cells in response to stimulation by alloantigen-presenting cells (FIG. 3A).

As shown in FIG. 4A, CD45RA⁺CD25⁺CD4⁺ T cells proliferated at high efficiency in the presence of IL-2 when stimulated with anti-CD3/CD28 antibody conjugate beads every 10-14 days; the cell number in the culture product increased about 3000 times in 50 days after the start of cultivation. After the cell number increased about 100-150 times, the beads were removed and the cultivation was performed in the presence of 100 U/ml IL-2 for 2 days. The obtained CD45RA⁺CD25⁺CD4⁺ T cell lines strongly suppressed the proliferation of CD4⁺ T cells in response to stimulation by alloantigen-presenting cells (FIG. 3B). However, when expansion was further continued, the proliferation suppressing function of the T cell line weakened (FIG. 4B, not treated with rapamycin).

Here, when rapamycin was added from the start of cultivation, the proliferation of CD45RA⁺CD25⁺CD4⁺ T cells was suppressed, and the cell number in the culture product increased only about 20 times in 50 days after the start of cultivation (FIG. 4A, day 0). After cultivation in the presence of rapamycin for 50 days, the beads and rapamycin were removed from the culture product, the CD45RA⁺CD25⁺CD4⁺ T cell line in the culture product was further cultured in the presence of 100 U/ml IL-2 for 2 days, and the proliferation suppressive function of the CD45RA⁺CD25⁺CD4⁺ T cell line obtained was examined. As a result, the T cell line strongly suppressed the proliferation of CD4⁺ T cells in response to stimulation by alloantigen-presenting cells (FIG. 4B, day 0).

After CD45RA⁺CD25⁺CD4⁺ T cells were proliferated in the absence of rapamycin, the effects of rapamycin added to the medium on the proliferation of the CD45RA⁺CD25⁺CD4⁺ T cell line and proliferation suppressive function on CD4⁺ T cells were investigated. When rapamycin was added 7 days or 39 days after the start of cultivation, the proliferation of the CD45RA⁺CD25⁺CD4⁺ T cell line was suppressed after the time of addition of rapamycin (FIG. 4A, day 7, day 39). When rapamycin was added 7 days after the start of cultivation, the cell number in the culture product increased only about 60 times in 50 days after the start of cultivation. Meanwhile, when rapamycin was added 39 days after the start of cultivation, the cell number in the culture product increased about 700 times in 50 days after the start of cultivation. Fifty days after the start of cultivation, the beads and rapamycin were removed from the culture product as described above, the CD45RA⁺CD25⁺CD4⁺ T cell line in the culture product was further cultured in the presence of 100 U/ml IL-2 for 2 days, and the proliferation suppressive function of the CD45RA⁺CD25⁺CD4⁺ T cell line obtained was examined. As a result, the T cell line strongly suppressed the proliferation of CD4⁺ T cells in response to stimulation by alloantigen-presenting cells (FIG. 4B, day 7, day 39).

From these results, it was demonstrated that by additions of a rapamycin compound, the CD45RA⁺CD25⁺CD4⁺ T cell line could be expanded while keeping the immunosuppressive function thereof at a high level. It was suggested that cell proliferation and maintenance of immunomodulatory function are more sufficiently achieved when a rapamycin compound is added in the late stage (day 39) of cultivation than in the initial stage (days 0 and 7) of cultivation.

### (Functional characteristics of expanded CD45RA⁺CD25⁻CD4⁺ T cells)

CD45RA⁺CD25⁻CD4⁺ T cells proliferated 1000 times or more in number in about 35 days when stimulated with anti-CD3/CD28 antibody conjugate beads in the presence of IL-2 (FIG. 5A, not treated). After expansion in the absence of rapamycin for about 35 days, the beads were removed, the cells were further cultured in the presence of 100 U/ml IL-2 for 2 days; the T cell line obtained did not suppress the proliferation of CD4⁺ T cells in response to stimulation by alloantigen-presenting cells (FIG. 5B, not treated).

Meanwhile, when rapamycin was added from the start of cultivation, the proliferation of CD45RA⁺CD25⁻CD4⁺ T cells was slightly suppressed compared with cultivation in the absence of rapamycin, but the cell number in the culture product increased about 700 times in about 35 days after the start of cultivation (FIG. 5A, day 0). After about 35 days of cultivation in the presence of rapamycin, the beads and rapamycin were removed from the culture product, and the cells were further cultured in the presence of 100 U/ml IL-2 for 2 days; the T cell line obtained strongly suppressed the proliferation of CD4⁺ T cells in response to stimulation by alloantigen-presenting cells (FIG. 5B, day 0).

When rapamycin was added to the culture product after CD45RA⁺CD25⁻CD4⁺ T cells were expanded in the absence of rapamycin, the effects of rapamycin on T cell line proliferation and proliferation suppressive function on CD4⁺ T cell were investigated. Even when rapamycin was added 19 days after the start of cultivation, there was almost no influence on the proliferation of the T cell line (FIG. 5A, day 19). About 35 days after the start of cultivation, the beads and rapamycin were removed from the culture product as described above, and the cells were further cultured in the presence of 100 U/ml IL-2 for 2 days; the T cell line obtained strongly suppressed the proliferation of CD4⁺ T cells in response to stimulation by alloantigen-presenting cells (FIG. 5B, day 19).

From these results, it was demonstrated that regulatory T cells could be induced by culturing CD25⁻CD4⁺ T cells having a naive phenotype in the presence of a rapamycin compound. It was also shown that by using a rapamycin compound, irrespective of the presence or absence of CD25 expression, regulatory T cells could be produced from CD4⁺T cells having a naive phenotype.

### [Example 2]

### [1] Methods

### (Cell culture)

CD45RA⁺CD25⁺CD4⁺ T cells or CD45RA⁺CD25⁻CD4⁺ T cells in human peripheral blood separated in the same manner as Example 1 (5 x 10^4 cells/well) were stimulated with anti-human CD3 and CD28 conjugate beads in a ratio of 4 beads per cell in a 96-well round-bottomed culture plate containing 200 µl per well of a proliferation medium (AlyS505N (produced by Cell Science & Technology Institute) supplemented with 2% human AB serum) in the presence of human IL-2 (1000 U/ml, produced by Cell Science & Technology Institute). Rapamycin (20 nM, produced by Sigma) was added to the medium 0 or 40 days after the start of cultivation. Every 3 to 4 days, half of the medium was replaced with a fresh proliferation medium containing IL-2. If necessary, the cells were separated and repeatedly re-stimulated with anti-CD3/CD28 antibody conjugate beads every 10 to 14 days. Fifty days after the start of cultivation, the cells were collected and used for FACS analysis.

### (FACS analysis)

Expanded T cell lines were incubated with various antibodies for 30 minutes at 4°C in the dark, washed, and analyzed using BD FACSAria cell sorter and FACSDiVA software (Becton Dickinson). The antibodies and staining conditions used were the same as Example 1.

### [2] Results

Results of flowcytometry analysis are shown in FIG. 6.

The T cell line obtained using CD45RA⁺CD25⁺CD4⁺ T cells in human peripheral blood with rapamycin added to the medium from day 40, like naturally occurring regulatory T cells in human peripheral blood (CD45RA-CD25^{high+}CD4⁺ T cells in peripheral blood), were GITR⁺CTLA4⁺. The CD45RB expression in the T cell line was higher (about 4 times) than that by CD45RA⁻CD25^{high+}CD4⁺ T cells in peripheral blood (CD45RB^{high+}). The CD25 expression in the T cell line was nearly the same (about 0.8 times) as that in CD45RA⁻CD25^{high+}CD4⁺ T cells in peripheral blood (CD25^{high+}), and the CD62L expression was lower (about 1/6 times) than that in CD45RA⁻CD25^{high+}CD4⁺ T cells in peripheral blood (CD62L^{low+}). The CD45RA and CD45RO expression intensities in the T cell line exhibited intermediate values between those of CD45RA⁺CD25⁺CD4⁺ T cells at the start of cultivation and those of CD45RA⁻CD25^{high+}CD4⁺ T cells in peripheral blood (CD45RA^{mid+}CD45RO^{mid+}). The CD45RA expression was about 1/6 times that of CD45RA⁺CD25⁺CD4⁺ T cells in peripheral blood, and the CD45RO expression was about 1/50 times that of CD45RA⁻CD25^{high+}CD4⁺ T cells in peripheral blood.

The phenotype of the T cell line obtained using CD45RA⁺CD25⁺CD4⁺ T cells in human peripheral blood with rapamycin added to the medium from day 0 was the same as the phenotype of the T cell line obtained with rapamycin added from day 40, except that the former phenotype was negative for CD45RO (CD45RO⁻).

The T cell line obtained using CD45RA⁺CD25⁻CD4⁺ T cells in human peripheral blood with rapamycin added to the medium from day 40, as with the use of CD45RA⁺CD25⁺CD4⁺ T cells in peripheral blood, had the GITR⁺CTLA4⁺CD45RB^{high+} phenotype. The CD25 expression in the T cell line was lower (about 1/5 times) than that in CD45RA⁻CD25^{high+}CD4⁺ T cells in peripheral blood (CD25^{low+}), and the CD62L expression was nearly the same (about 1/3 times) as that by CD45RA⁻CD25^{high+}CD4⁺ T cells in peripheral blood (CD62L^{high+}). The CD45RA expression intensity of the T cell line was nearly the same (about 1 time) as that of CD45RA⁺CD25⁻CD4⁺ T cells at the start of cultivation (CD45RA^{high+}). The T cell line was negative for CD45RO (CD45RO⁻).

Hence, it was demonstrated that regulatory T cells that can be produced by the method of the present invention have a phenotype different from that of naturally occurring regulatory T cells in periphery (CD45RA⁻CD25^{high+}CD4⁺ T cells in peripheral blood), and that the phenotype is variable according to the kind of CD4⁺ T cells having a naive phenotype used in the method of the present invention (e.g., presence or absence of CD25 expression and the like) and the timing of addition of rapamycin to the medium.

### [Example 3]

### [1] Methods

### (Cell culture)

CD45RA⁺CD25⁺CD4⁺ T cells or CD45RA⁺CD25⁻CD4⁺ T cells in human peripheral blood separated in the same manner as Example 1 (5 x 10^4 cells/well) were stimulated with anti-human CD3 and CD28 conjugate beads in a ratio of 4 beads per cell in a 96-well round-bottomed culture plate containing 200 µl per well of a proliferation medium (AlyS505N (produced by Cell Science & Technology Institute) supplemented with 2% human AB serum) in the presence of human IL-2 (1000 U/ml, produced by Cell Science & Technology Institute). Rapamycin (20 nM, produced by Sigma) was added to the medium 40 days after the start of cultivation. Every 3 to 4 days, half of the medium was replaced with a fresh proliferation medium containing IL-2. If necessary, the cells were separated and repeatedly re-stimulated with anti-CD3/CD28 antibody conjugate beads every 10 to 14 days. Fifty days after the start of cultivation, the cells were collected and used for functional assay.

### (Mixed lymphocyte reaction (MLR))

Total CD4⁺ T cells in peripheral blood were isolated in the same manner as Example 1. The indicated numbers of respective T cell lines expanded were stimulated with 5×10⁴ isolated peripheral blood total CD4⁺ T cells and 1×10⁵ radiated (40 Gy) allogenic PBMCs, along with 10 ng/ml anti-human CD3 antibody (OKT-3), in a proliferation medium containing 10% FBS in a round-bottomed 96 well microplate. For a trans-well experiment, 1×10⁶ freshly-isolated total CD4⁺ T cells and 1×10⁶ cells of each T cell line were co-cultured, or total CD4⁺ T cells and each T cell line were cultured separately on the bottom and upper portion of the chamber along with allogenic PBMCs (3×10⁶ cells). The culture product was incubated for 4 days, and labeled with [3H] thymidine during the last 16 hours. The culture product was collected, and [3H] thymidine uptake was measured using 1450 Microbeta TriLux (Wallac).

### [2] Results

Under conditions that allow the contact of T cell lines and total CD4⁺ T cells, the CD45RA⁺CD25⁺CD4⁺ T cell line and the CD45RA⁺CD25⁻CD4⁺ T cell line both strongly suppressed the proliferation of total CD4⁺ T cells (FIG. 7). However, when the contact of T cell lines and total CD4⁺ T cells was blocked with the trans-well, the suppression of the proliferation of total CD4⁺ T cells by the CD45RA⁺CD25⁺CD4⁺ T cell line was interrupted, whereas the CD45RA⁺CD25⁻CD4⁺ T cell line continued to suppress the proliferation of total CD4⁺ T cells.

Thus, it was demonstrated that the regulatory T cells obtained with the use of CD25⁺CD4⁺ T cells having a naive phenotype in the method of the present invention exhibited immunosuppressive function depending on cell contact, whereas the regulatory T cells obtained with the use of CD25⁻CD4⁺ T cells having a naive phenotype exhibited immunosuppressive function without dependence on cell contact.

### [Example 4]

### [1] Methods

### (Cell culture)

CD45RA⁺CD25⁺CD4⁺ T cells or CD45RA⁺CD25⁻CD4⁺ T cells in human peripheral blood (5 x 10^4 cells/well) were stimulated with 1 x 10^5 cells/well radiated (40 Gy) allogenic PBMCs in a 96-well round-bottomed culture plate containing 200 µl per well of a proliferation medium (AlyS505N (produced by Cell Science & Technology Institute) supplemented with 2% human AB serum) in the presence of human IL-2 (1000 U/ml, produced by Cell Science & Technology Institute).

Everolimus (100 nM or 1000 nM, produced by Novartis Pharma) was added to the medium from the start of cultivation. The everolimus was purified as follows: Certican 0.25 mg tablet (produced by Novartis Pharma) was milled and dissolved in acetone. Then, the solution was filtered, the filtrate was evaporated while rotating, and the precipitate was vacuum-dried. The extract obtained was dissolved in DMF and separated by HPLC (column C18 4 µm, 150X2.1 mm). After separation, the everolimus was freeze-dried and purified.

Every 3 to 4 days, half of the medium was replaced with a fresh proliferation medium containing IL-2. If necessary, the cells were separated and repeatedly re-stimulated with radiated (40 Gy) allogenic PBMC every 10 to 14 days. Fifty days after the start of cultivation, the cells were collected and used for functional assay.

### (Suppression assay)

The indicated number of cells of the expanded CD45RA⁺CD25⁺CD4⁺ T cell line or expanded CD45RA⁺CD25⁻CD4⁺ T cell line, alone or along with autologous responder CD4⁺ T cells (5 x 10^4 cells/well), were stimulated with radiated (40 Gy) PBMCs (1 x 10^5 cells/well) obtained from the same individual as the allogenic PBMCs used for the cultivation, in a proliferation medium in a round-bottomed 96-well microplate. The culture product was incubated for 7 days, and labeled with [³H] thymidine during the last 16 hours (2 µCi/well). Subsequently, the cells were recovered, and [³H] thymidine uptake was measured using 1450 Microbeta TriLux (Wallac).

### [2] Results

### (Effects of everolimus on the function of CD45RA⁺CD25⁺CD4⁺ T cell line)

By adding everolimus (1000 nM), the proliferation of CD45RA⁺CD25⁺CD4⁺ T cells was suppressed (FIG. 8A). However, the CD45RA⁺CD25⁺CD4⁺ T cell line obtained by cultivation in the presence of everolimus, compared with controls, more strongly suppressed the proliferation of CD4⁺ T cells in response to stimulation by alloantigen-presenting cells (FIG. 9).

Thus, it was demonstrated that as with rapamycin, by adding everolimus, the CD45RA⁺CD25⁺CD4⁺ T cell line could be expanded while keeping the immunosuppressive function thereof at a high level.

### (Effects of everolimus on the function of CD45RA⁺CD25⁻CD4⁺ T cell line)

Surprisingly, by adding everolimus (100 nM or 1000 nM), the proliferation of CD45RA⁺CD25⁻CD4⁺ T cells was enhanced (FIG. 8B). The CD45RA⁺CD25⁻CD4⁺ T cell line obtained by everolimus-free cultivation weakly suppressed the proliferation of CD4⁺ T cells in response to stimulation by alloantigen-presenting cells (FIG. 10, control). Meanwhile, the CD45RA⁺CD25⁻CD4⁺ T cell line obtained by cultivation in the presence of everolimus more strongly suppressed the proliferation of CD4⁺ T cells in response to stimulation by alloantigen-presenting cells compared with the control (FIG. 10).

Thus, it was demonstrated that regulatory T cells could be induced by culturing CD25⁻CD4⁺ T cells having a naive phenotype (CD45RA⁺) in the presence of everolimus, as with rapamycin. In particular, everolimus was excellently effective in enhancing the proliferation of the CD45RA⁺CD25⁻CD4⁺ T cell line.

It was also demonstrated that as with rapamycin, by using everolimus, irrespective of the presence or absence of CD25 expression, regulatory T cells could be produced from CD4⁺T cells having a naive phenotype (CD45RA⁺).

### [Example 5]

### (Increases in the number of CD45RA⁺CD25⁺CD4⁺ T cells in peripheral blood of patients undergoing liver transplantation from living donor)

(Method) Before surgery and over time after surgery for liver transplantation from a living donor (orthotropic liver transplantation) (1 month, 3 months, 6 months, 1 year, 2 years; 10 children (age 0 to 18 years) and 10 adults (age 39 to 64 years) at each time point), blood was drawn cross sectionally, and the ratio (%) of the CD45RA⁺CD25⁺CD4⁺ T cell fraction to total lymphocyte count was analyzed using a flowcytometer. The antibodies used were APC-anti-CD4 antibody, PE-anti-CD25 antibody, and FITC-anti-CD45RA antibody at the optimum doses previously determined by titration.
(Results) The CD45RA⁺CD25⁺CD4⁺ T cells fraction increased remarkably after liver transplantation in the children, reaching a peak at 3 months after surgery. Meanwhile, in the adults, the same fraction did not show a remarkable increase as found in the children, but the ratio of the cell fraction increased gradually (FIG. 11).

### Industrial Applicability

By using the screening method of the present invention, a compound capable of inducing regulatory T cells can be obtained. The compound is useful as an immunosuppressive drug based on the new mechanism of inducing regulatory T cells, or as a seed for the development thereof.

In addition, using the method of the present invention, a mammalian regulatory T cell can be efficiently produced in vitro. The regulatory T cell produced by the method is useful as an immunomodulator for the prophylaxis or treatment of rejection reaction in organ transplantation, allergic disease, autoimmune disease, graft-versus-host disease (GVHD), infertility and the like.

This application is based on a patent application No. 2005-289224 filed in Japan (filing date: September 30, 2005), the content of which is incorporated in full herein by this reference.

## Claims

1. A screening method for a compound capable of inducing regulatory T cells, comprising the following steps:
(1) a step for culturing CD4⁺ T cells having at least one naive phenotype selected from the group consisting of (1) CD45RA⁺,
(2) CD45RO^{low+} or CD45RO⁻, (3) CD45RO^{high+} and (4) CD38⁺ in the presence of a test compound, and isolating T cells from the culture product;
(2) a step for evaluating the immunosuppressive function of the T cells isolated in the step (1);
(3) a step for obtaining the foregoing test compound as a compound capable of inducing regulatory T cells if the evaluation in the step (2) shows that the T cells isolated in the step (1) have the immunosuppressive function.

2. The method of claim 1, wherein the naïve phenotype is CD45RA⁺.

3. The method of claim 1, wherein the CD4⁺ T cells are CD25⁺.

4. The method of claim 1, wherein the CD4⁺ T cells are CD25⁻.

5. The method of claim 1, wherein the CD4⁺ T cells are of primate origin.

6. The method of claim 1, wherein the CD4⁺ T cells are cultured in the presence of an antigen.

7. The method of claim 1, wherein the CD4⁺ T cells are cultured in the presence of a T cell growth factor.

8. The method of claim 1, wherein the test compound is an immunosuppressive compound or a derivative thereof.

9. A screening method for a compound capable of increasing the efficiency of production of regulatory T cells, comprising the following steps:
(1) a step for culturing CD25⁺CD4⁺ T cells having at least one naive phenotype selected from the group consisting of (1) CD45RA⁺, (2) CD45RO^{low+} or CD45RO⁻, (3) CD45RO^{high+} and (4) CD38⁺ in the presence of a test compound to obtain regulatory T cells;
(2) a step for evaluating the number and/or immunosuppressive function of the regulatory T cells obtained in the step (1);
(3) a step for comparing the number and/or immunosuppressive function of the regulatory T cells evaluated in the step (2) with the number and/or immunosuppressive function of regulatory T cells obtained by culturing CD25⁺CD4⁺ T cells having at least one naive phenotype selected from the group consisting of (1) CD45RA⁺, (2) CD45RO^{low+} or CD45RO⁻, (3) CD45RO^{high+} and (4) CD38⁺ in the absence of the test compound;
(4) a step for obtaining a test compound that has increased the number and/or immunosuppressive function of regulatory T cells as a compound capable of increasing the efficiency of production of regulatory T cells.

10. The method of claim 9, wherein the naive phenotype in the steps (1) and (3) is CD45RA⁺.

11. The method of claim 9, wherein the CD25⁺CD4⁺ T cells in the steps (1) and (3) are of primate origin.

12. The method of claim 9, wherein the CD25⁺CD4⁺ T cells are cultured in the presence of an antigen in the steps (1) and (3).

13. The method of claim 9, wherein the CD25⁺CD4⁺ T cells are cultured in the presence of a T cell proliferation factor in the steps (1) and (3).

14. The method of claim 9, wherein the test compound is an immunosuppressive compound or a derivative thereof.

15. A method of producing regulatory T cells, comprising culturing CD4⁺ T cells having at least one naive phenotype selected from the group consisting of (1) CD45RA⁺, (2) CD45RO^{low+} or CD45RO⁻, (3) CD45RO^{high+} and (4) CD38⁺ in the presence of a rapamycin compound to obtain regulatory T cells.

16. The method of claim 15, wherein the naive phenotype is CD45RA⁺.

17. The method of claim 15, wherein the CD4⁺ T cells are CD25⁺.

18. The method of claim 15, wherein the CD4⁺ T cells are CD25⁻.

19. The method of claim 15, wherein the CD4⁺ T cells are of primate origin.

20. The method of claim 15, wherein the CD4⁺ T cells are cultured in the presence of an antigen.

21. The method of claim 15, wherein the CD4⁺ T cells are cultured in the presence of a T cell growth factor.

22. The method of claim 15, wherein the rapamycin compound is added to the medium 1 week after the start of cultivation or later.

23. The method of claim 17, wherein the obtainable regulatory T cells have immunosuppressive function that depends on cell contact.

24. The method of claim 18, wherein the obtainable regulatory T cells have immunosuppressive function that does not depend on cell contact.

25. The method of claim 15, wherein the rapamycin compound is rapamycin.

26. The method of claim 15, wherein the rapamycin compound is everolimus.

27. A method of maintaining the immunosuppressive function of regulatory T cells in producing regulatory T cells by culturing CD25⁺CD4⁺ T cells having at least one naive phenotype selected from the group consisting of (1) CD45RA⁺, (2) CD45RO^{low+} or CD45RO⁻, (3) CD45RO^{high+} and (4) CD38⁺ to obtain regulatory T cells, which comprise culturing the CD25⁺CD4⁺ T cells in the presence of a rapamycin compound.

28. A method of inducing regulatory T cells, comprising culturing CD25⁻CD4⁺ T cells having at least one naive phenotype selected from the group consisting of (1) CD45RA⁺, (2) CD45RO^{low+} or CD45RO⁻, (3) CD45RO^{high+} and (4) CD38⁺ in the presence of a rapamycin compound.

29. Regulatory T cells that can be obtained by the method according to claim 15, having at least one phenotype selected from one of the groups (A) to (C) below:
(A) CD45RO^{mid+}, CD45RA^{mid+}, CD62L^{low+} and CD25^{high+}
(B) CD45RO⁻, CD45RA^{mid+}, CD62L^{low+} and CD25^{high+}
(C) CD45RO⁻, CD45RA^{high+}, CD62L^{high+} and CD25^{low+}.

30. Regulatory T cells having at least one phenotype selected from one of the groups (B) and (C) below:
(B) CD45RO⁻, CD45RA^{mid+}, CD62L^{low+} and CD25^{high+}
(C) CD45RO⁻, CD45RA^{high+}, CD62L^{high+} and CD25^{low+}.

31. An immunomodulator containing the regulatory T cells of claim 29 or 30 as an active ingredient.

32. The agent of claim 31, which is to be used for immunosuppression.

33. A method of producing an immunomodulator, comprising the following steps:
(1) a step for culturing CD4⁺ T cells having at least one naive phenotype selected from the group consisting of (1) CD45RA⁺,
(2) CD45RO^{low+} or CD45RO⁻, (3) CD45RO^{high+} and (4) CD38⁺ in the presence of a rapamycin compound to obtain regulatory T cells;
(2) a step for mixing the regulatory T cells with a pharmaceutically acceptable carrier to give an immunomodulator.

34. The method of claim 33, wherein the immunomodulator is to be used for immunosuppression.

35. An agent for inducing regulatory T cells from CD4⁺ T cells having at least one naive phenotype selected from the group consisting of (1) CD45RA⁺, (2) CD45RO^{low+} or CD45RO⁻, (3) CD45RO^{high+} and (4) CD38⁺, comprising a rapamycin compound.

36. The agent of claim 35, wherein the CD4⁺ T cells are CD25⁻.

37. An agent for maintaining the immunosuppressive function of regulatory T cells in producing regulatory T cells by culturing CD4⁺ T cells having at least one naive phenotype selected from the group consisting of (1) CD45RA⁺, (2) CD45RO^{low+} or CD45RO⁻, (3) CD45RO^{high+} and (4) CD38⁺ to obtain regulatory T cells, comprising a rapamycin compound.

38. The agent of claim 37, wherein the CD4⁺ T cells are CD25⁺.

39. A kit for production of regulatory T cells, comprising an antibody for preparing CD4⁺ T cells having at least one naive phenotype selected from the group consisting of (1) CD45RA⁺, (2) CD45RO^{low+} or CD45RO⁻, (3) CD45RO^{high+} and (4) CD38⁺, and a rapamycin compound.

40. A method of inducing CD25⁺CD4⁺ T cells having a naive phenotype in vivo, comprising the following steps:
(1) a step for performing liver transplantation on a non-human mammal;
(2) a step for confirming the induction of CD25⁺CD4⁺ T cells having a naïve phenotype in peripheral tissue of the foregoing non-human mammal.

41. A screening method for a compound capable of increasing the number of CD25⁺CD4⁺ T cells having a naive phenotype in vivo, comprising the following steps:
(1) a step for performing liver transplantation on a non-human mammal;
(2) a step for administering a test compound to the mammal of the step (1);
(3) a step for evaluating the number of CD25⁺CD4⁺ T cells having a naive phenotype in peripheral tissue of the mammal of the step (2);
(4) a step for comparing the number of CD25⁺CD4⁺ T cells having a naive phenotype evaluated in the step (3) with the number of CD25⁺CD4⁺ T cells having a naïve phenotype in peripheral tissue of a non-human mammal that has undergone liver transplantation, but has not received the test compound;
(5) a step for selecting a compound that has increased the number of CD25⁺CD4⁺ T cells having a naive phenotype in the peripheral tissue.

42. A use of the regulatory T cells of claim 29 or 30 for production of an immunomodulator.

43. A use of a rapamycin compound for producing an agent for inducing regulatory T cells from CD4⁺ T cells having at least one naive phenotype selected from the group consisting of (1) CD45RA⁺, (2) CD45RO^{low+} or CD45RO⁻, (3) CD45RO^{high+} and (4) CD38⁺.

44. A use of a rapamycin compound for producing an agent for maintaining the immunosuppressive function of regulatory T cells in producing regulatory T cells by culturing CD4⁺ T cells having at least one naive phenotype selected from the group consisting of (1) CD45RA⁺, (2) CD45RO^{low+} or CD45RO⁻, (3) CD45RO^{high+} and (4) CD38⁺ to obtain regulatory T cells.
